# EUROPEAN PATENT SPECIFICATION

(11) **EP 3 650 543 B1**
(45) Date of publication and mention of the grant of the patent: **07.06.2023**
(21) Application number: 18828987.0
(22) Date of filing: 05.07.2018
(51) Int. Cl.: C12N 15/10

(54) **DNA PRODUCTION METHOD AND DNA FRAGMENT JOINING KIT**
DNA-PRODUKTIONSVERFAHREN UND KIT ZUR VERBINDUNG VON DNA-FRAGMENTEN
MÉTHODE DE PRODUCTION D'ADN ET KIT D'ASSEMBLAGE DE FRAGMENT D'ADN

(30) Priority: 05.07.2017 JP 2017132084; 01.12.2017 JP 2017231732
(43) Date of publication of application: 13.05.2020
(73) Proprietor: OriCiro Genomics, Inc., Bunkyo-ku Tokyo 113-8485 (JP)
(72) Inventor: SUETSUGU Masayuki, Tokyo 171-8501 (JP); KURATA Tatsuaki, Tokyo 171-8501 (JP)
(74) Representative: Marks & Clerk LLP
(86) International application number: PCT/JP2018/025528
(87) International publication number: WO 2019/009361

(56) References cited:
- WO-A1-2016/080424
- WO-A2-2008/035205
- JP-A- 2016 077 180
- US-A1- 2007 037 196
- BAOGONG ZHU ET AL: "In-Fusion(TM) assembly: seamless engineering of multidomain fusion proteins, modular vectors, and mutations", BIOTECHNIQUES, vol. 43, no. 3, 1 September 2007 (2007-09-01), pages 354-359, XP055227187, US ISSN: 0736-6205, DOI: 10.2144/000112536
- CHANGLIN FU ET AL: "Hot Fusion: An Efficient Method to Clone Multiple DNA Fragments as Well as Inverted Repeats without Ligase", PLOS ONE, vol. 9, no. 12, 31 December 2014 (2014-12-31), page e115318, XP055418178, DOI: 10.1371/journal.pone.0115318
- DANIEL G GIBSON ET AL: "Enzymatic assembly of DNA molecules up to several hundred kilobases", NATURE METHODS, vol. 6, no. 5, 12 April 2009 (2009-04-12), pages 343-345, XP055224105, New York ISSN: 1548-7091, DOI: 10.1038/nmeth.1318

## Description

### [Technical Field]

The present invention relates to a method for producing linear or circular DNA by joining two or more types of DNA fragments to each other at regions having homologous base sequences, and a DNA fragment-joining kit used in the method.

Priority is claimed on Japanese Patent Application No. 2017-132084, filed on July 5, 2017, and Japanese Patent Application No. 2017-231732, filed on December 1 2017

### [Background Art]

There is a method of producing linear or circular double-stranded DNA by joining a plurality of linear double-stranded DNA fragments. By this method, a longer double-stranded DNA that is difficult to synthesize by chemical synthesis can be obtained. Methods for joining linear double-stranded DNA fragments mainly include the Infusion method (refer to Patent Literature 1) and the Gibson Assembly method (refer to Patent Literature 2 and Patent Literature 3).

The Infusion method is a method in which a joining reaction is performed using an Infusion enzyme having a function of recognizing a homologous sequence of the terminal 15 bases of each double-stranded DNA fragment and fusing them. Specifically, first, a homologous region consisting of the same base sequence is added to the ends of the two double-stranded DNA fragments to be joined using PCR. Two double-stranded DNA fragments added with a 15-base homologous region at both ends are joined by mixing with Infusion enzyme and incubating.

On the other hand, in the Gibson Assembly method, first, the distal region of the first DNA molecule and the proximal region of the second DNA molecule are digested with an enzyme having exonuclease activity. As a result, the respective homologous regions (regions with identical sequences that are long enough to specifically hybridize to each other) are brought into a single-stranded state. Next, after both are specifically annealed and joined, gaps and nicks are repaired to obtain a complete double-stranded DNA joined body. For example, Patent Literature 2 discloses the following: 1.6 kbp and 1.4 kbp double-stranded DNA fragments were made into a single stranded state by digesting with T4 DNA polymerase having exonuclease activity. The resulting two single-stranded DNAs were joined in the presence of RecA, dNTPs and DNA ligase were added, the gap was filled with the polymerase activity of this T4 DNA polymerase to repair nicks, and a 3 kbp double-stranded DNA was obtained.

In addition, Patent Literature 4 describes a method of joining a plurality of linear double-stranded DNA fragments using RecA. The method includes a step of incubating a plurality of linear double-stranded DNA fragments with a DNA ligase in the presence of a RecA family recombinase or a protein having recombination activity, thereby producing a linear double-stranded DNA in which a plurality of linear double-stranded DNA fragments are joined in series. In this method, by using a RecA family recombination enzyme or the like, binding of both ends in the linear double-stranded DNA fragment is suppressed, and binding between the ends of the linear double-stranded DNA fragments is promoted.

### [Citation List]

### [Patent Literature]

[Patent Literature 1] U.S. Patent No. 7,575,860
[Patent Literature 2] U.S. Patent No. 7,776,532
[Patent Literature 3] U.S. Patent No. 8,968,999
[Patent Literature 4] Japanese Unexamined Patent Application, First Publication No. 2016-077180

### [Summary of Invention]

### [Technical Problem]

The Infusion method and Gibson Assembly method can join two linear DNA fragments without any problem. However, in these methods, there is a problem in that as the number of joined fragments increases, the joining efficiency decreases, and the joined body of interest cannot be obtained.

An object of the present invention is to provide a method for producing linear or circular DNA by joining two or more types of DNA fragments to each other at regions having homologous base sequences, and a DNA fragment-joining kit used in the method.

### [Solution to Problem]

As a result of extensive research, the present inventors have found that two or more types of DNA can be efficiently joined by using RecA family recombinase and exonuclease, thereby completing the present invention.

A DNA production method and DNA fragment-joining kit according to the present invention are defined by the claims.

### [Advantageous Effects of Invention]

By the DNA production method according to the present invention, a plurality of DNA fragments can be efficiently joined, and as a result, linear or circular DNA can be obtained.

By the DNA fragment-joining kit, the DNA production method can be performed more simply, and DNA fragments can be efficiently joined together.

### [Brief Description of the Drawings]

Fig. 1 is a diagram schematically showing an embodiment in which linear double-stranded DNA fragments are joined to each other using the principle of the DNA production method according to the present invention.
Fig. 2 is a stained image of bands separated by agarose gel electrophoresis of the reaction solution obtained by joining reaction of 7 fragments in Example 1.
Fig. 3 is a stained image of bands separated by agarose gel electrophoresis of the reaction solution obtained by joining reaction of 5 fragments in Example 2.
Fig. 4 is a stained image of bands separated by agarose gel electrophoresis of the reaction solution obtained by joining reaction of 5 fragments in Example 3.
Fig. 5 is a stained image of bands separated by agarose gel electrophoresis of the reaction solution obtained by joining reaction of 7 fragments in Example 4.
Fig. 6 is a stained image of bands separated by agarose gel electrophoresis of the reaction solution obtained by joining reaction of 5 fragments in Example 5.
Fig. 7 is a stained image of bands separated by agarose gel electrophoresis of the reaction solution obtained by joining reaction of 7 fragments in Example 5.
Fig. 8 is a stained image of bands separated by agarose gel electrophoresis of the reaction solution obtained by joining reaction of 7 fragments in Example 6.
Fig. 9 is a stained image of bands separated by agarose gel electrophoresis of the reaction solution obtained by joining reaction of 7 fragments in Example 7.
Fig. 10 is a stained image of bands separated by agarose gel electrophoresis of the reaction solution obtained by joining reaction of 20-49 fragments in Example 8.
Fig. 11 is a stained image of bands separated by agarose gel electrophoresis of the reaction solution obtained by joining reaction of 25 fragments in Example 9.
Fig. 12 shows (a) a stained image of bands separated by agarose gel electrophoresis of the reaction solution obtained by joining reaction of 21 or 26 fragments, and (b) a stained image of bands separated by agarose gel electrophoresis of the reaction mixture obtained by further RCR amplification after the joining reaction in Example 10.
Fig. 13 shows (a) a stained image of bands separated by agarose gel electrophoresis of the reaction solution obtained by joining reaction of 26 fragments, and (b) a stained image of bands separated by agarose gel electrophoresis of the reaction mixture obtained by further RCR amplification after the joining reaction in Example 11.
Fig. 14 shows (a) a stained image of bands separated by agarose gel electrophoresis of the reaction solution obtained by joining reaction of 26 or 36 fragments, and (b) a stained image of bands separated by agarose gel electrophoresis of the reaction mixture obtained by further RCR amplification after the joining reaction in Example 12.
Fig. 15 shows (a) a stained image of bands separated by agarose gel electrophoresis of the reaction solution after joining reaction of 26 fragments by the joining method (RA) according to the present invention and NEB method, and (b) a stained image of bands separated by agarose gel electrophoresis of the reaction mixture obtained by further RCR amplification after the joining reaction in Example 13.
Fig. 16 is a stained image of bands separated by agarose gel electrophoresis of the reaction mixture obtained by joining the Xba I digest of E. coli genomic DNA with the fragment containing oriC into circular DNA, and then performing RCR amplification in Example 14.
Fig. 17 is a stained image of bands separated by agarose gel electrophoresis of the reaction solution obtained by joining reaction of 10 fragments in the reaction solution containing an ATP regeneration system consisting of creatine kinase (CK) and creatine phosphate (CP) in Example 15.
Fig. 18 is a stained image of bands separated by agarose gel electrophoresis of the reaction solution obtained by joining reaction of 10 fragments in the reaction solution containing the ATP regeneration system consisting of creatine kinase and creatine phosphate in Example 16.
Fig. 19 is a stained image of bands separated by agarose gel electrophoresis of the reaction solution obtained by joining reaction of 36 fragments in the reaction solution containing an ATP regeneration system consisting of pyruvate kinase (PK) and phosphoenolpyruvate (PEP) in Example 17.
Fig. 20 is a stained image of bands separated by agarose gel electrophoresis of the reaction solution obtained by joining reaction of 10 fragments in the reaction solution containing an ATP regeneration system consisting of polyphosphate kinase (PPK) and polyphosphate (PP) in Example 18.
Fig. 21 is a stained image of bands separated by agarose gel electrophoresis of the reaction solution obtained by joining reaction of 10 fragments in the reaction solution containing both exonuclease III and exonuclease I in Example 19.
Fig. 22 has (a) a stained image of bands separated by agarose gel electrophoresis of the reaction solution obtained by joining reaction of 36 fragments, and (b) a stained image of bands separated by agarose gel electrophoresis of the reaction mixture obtained by further RCR amplification after the joining reaction in Example 20.
Fig. 23 has (a) a stained image of bands separated by agarose gel electrophoresis of the reaction solution obtained by joining reaction of 50 or 36 fragments, and (b) a stained image of bands separated by agarose gel electrophoresis of the reaction mixture obtained by further RCR amplification after the joining reaction in Example 21.
Fig. 24 is a stained image of bands separated by agarose gel electrophoresis of the DNA extracted from the transformant in Example 21. The transformant was obtained by introducing into E. coli the DNA in the reaction solution obtained by further RCR amplification after the joining reaction of 50 fragments.
Fig. 25 is a stained image of bands separated by agarose gel electrophoresis of the enzymatic digest of amplified product obtained by RCR amplification of DNA extracted from the obtained transformant in Example 21.
Fig. 26 is a stained image of bands separated by agarose gel electrophoresis of the reaction solution obtained by joining reaction of 2 fragments in Example 22.
Fig. 27 is a stained image of bands separated by agarose gel electrophoresis of the reaction solution obtained by joining reaction of 10 fragments in the reaction solution containing exonuclease III, exonuclease I and exonuclease T in Example 23.
Fig. 28 is a stained image of bands separated by agarose gel electrophoresis of the reaction solution obtained by joining reaction of 10 fragments in the reaction solution containing UvsX, a bacteriophage RecA homolog, in Example 24.
Fig. 29 is a stained image of bands separated by agarose gel electrophoresis of the reaction solution obtained by joining reaction of 10 fragments in the reaction solution containing UvsX and UvsY in Example 25.

### Description of Embodiments

### <DNA production method >

In the DNA production method according to the present invention, DNA fragments with regions having base sequences that are homologous to each other (hereinafter, sometimes simply referred to as "homologous region") or regions having base sequences that are complementary to each other (hereinafter, sometimes simply referred to as "complementary region") are joined to each other at homologous regions or complementary regions to produce linear or circular DNA. Since the DNA production method according to the present invention performs a joining reaction in the presence of a protein having RecA family recombinase activity, the joining efficiency of the method is excellent.

In the present invention and the present specification, "the base sequences are homologous" means "the base sequences are identical", and "the base sequences are complementary" means "the base sequences are complementary to each other".

Specifically, in the method for producing DNA according to the present invention, a reaction solution containing two or more types of DNA fragments and a protein having RecA family recombinase activity (hereinafter sometimes referred to as "RecA family recombinase protein") is prepared, and the two or more types of DNA fragments are joined to each other at homologous regions or complementary regions in the reaction solution. This method produces linear or circular DNA. Hereinafter, linear or circular DNA in which two or more DNA fragments are linked may be referred to as "joined body".

In the method for producing DNA according to the present invention, a DNA fragment to be joined may be a linear double-stranded DNA fragment or a single-stranded DNA fragment. That is, linear double-stranded DNA fragments may be joined to each other, a linear double-stranded DNA fragment and a single-stranded DNA fragment may be joined to each other, or single-stranded DNA fragments may be joined to each other. One or more types of linear double-stranded DNA fragments and one or more types of single-stranded DNA fragments can be joined. When joining linear double-stranded DNA fragments or joining a linear double-stranded DNA fragment and a single-stranded DNA fragment, both fragments are joined to each other at the homologous region. When joining linear single-stranded DNA fragments, both fragments are joined to each other at the complementary region.

When at least one type of DNA fragment to be joined is a linear double-stranded DNA fragment in the DNA production method according to the present invention, the reaction solution further contains an exonuclease.

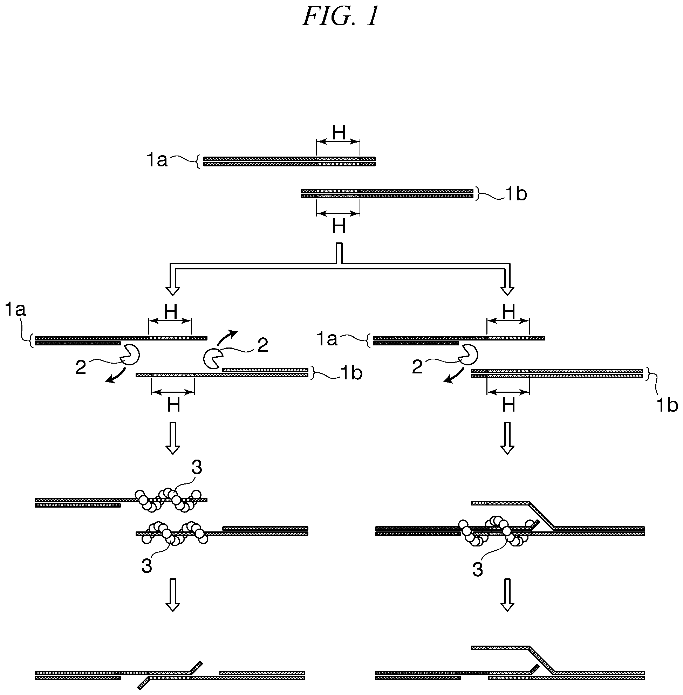

Fig. 1 shows a diagram schematically showing an embodiment in which linear double-stranded DNA fragments are joined to each other using the principle of the DNA production method according to the present invention. First, 3'→5' exonuclease 2 acts on a linear double-stranded DNA fragment 1a and a linear double-stranded DNA fragment 1b, both having a homologous region H to make the homologous region H into a single-stranded state. RecA family recombinase protein 3 acts on the homologous region H in the single-stranded state, and the homologous regions H that are complementary to each other are annealed to each other, whereby the linear double-stranded DNA fragment 1a and the linear double-stranded DNA fragment 1b are in a single-stranded state. As shown in the right figure of Fig. 1, cleaving by 3'→5' exonuclease 2 may be performed only on one of the linear double-stranded DNA fragment 1a or the linear double-stranded DNA fragment 1b. For example, the homologous region H of the linear double-stranded DNA fragment 1a in the single-stranded state acts to the homologous region H of the linear double-stranded DNA fragment 1b in the double-stranded state in the presence of RecA family recombinase protein 3, and both are linked.

When joining linear double-stranded DNA fragments or joining a linear double-stranded DNA fragment and a single-stranded DNA fragment in the DNA production method according to the present invention, the double-stranded DNA fragment is cleaved with exonuclease to make a homologous region into a single-strand state, and further a joining reaction is performed in the presence of RecA family recombinase protein. For this reason, the DNA production method according to the present invention according to the present invention is excellent in joining efficiency, and makes it possible to join multiple linear double-stranded DNA fragments in a single reaction that was difficult with the conventional techniques.

When joining linear single -stranded DNA fragments in the DNA production method according to the present invention, the RecA family recombinase protein rapidly forms a filament on each single-stranded DNA fragment, thereby inhibiting exonuclease digestion. Thereafter, the homologous regions H that are complementary to each other anneal to each other by the action of the RecA family recombinase protein, and both single-stranded DNA fragments are linked.

In the DNA production method according to the present invention, the number of DNA fragments to be joined is preferably 5 (5 fragments) or more, more preferably 7 (7 fragments) or more, further preferably 10 (10 fragments) or more, and may be 20 (20 fragments) or more. The upper limit of the number of DNA fragments to be joined in the DNA production method according to the present invention is not particularly limited. For example, numbers of up to 100 fragments can be linked. In the DNA production method according to the present invention, for example, about 50 linear double-stranded DNA fragments can be joined by optimizing reaction conditions and the like. In the DNA production method according to the present invention, it is possible to join to each other DNA fragments that are all different species. It is also possible to join to each other DNA fragments containing two or more DNA fragments of the same type.

Each of two or more types of DNA fragments to be joined in the present invention includes a homologous region or a complementary region for joining with at least one of other DNA fragments. When joining linear double-stranded DNA fragments or joining a linear double-stranded DNA fragment and a single-stranded DNA fragment in the DNA production method according to the present invention, first, one strand of the double-stranded DNA fragment is cleaved with exonuclease to make a homologous region into a single-stranded state. For this reason, the homologous region is preferably present at the end of the linear double-stranded DNA fragment. The homologous region may be present in the vicinity of the end. For example, the base on the terminal side of a linear double-stranded DNA fragment in the end of the homologous region is preferably within 300 bases from the terminal, more preferably within 100 bases, further preferably within 30 bases, and further more preferably within 10 bases. On the other hand, when joining linear single-stranded DNA fragments, exonuclease digestion is inhibited by the filament of RecA family recombinase proteins. So, the complementary region may be present in any part of the single-stranded DNA fragment.

The base sequences of the homologous regions or the complementary regions may be the same base sequence in all of the DNA fragments to be joined. It is preferable that the base sequences of homologous regions in DNA fragments to be joined be different for each type of DNA fragment in order to join them in a desired order. For example, in order to link the double-stranded DNA fragment A, the double-stranded DNA fragment B and the double-stranded DNA fragment C in this order, the homologous region a is placed both at the downstream end of the double-stranded DNA fragment A and at the upstream end of the double-stranded DNA fragment B, and the homologous region b is placed both at the downstream end of the double-stranded DNA fragment B and at the upstream end of the double-stranded DNA fragment C. The double-stranded DNA fragment A and the double-stranded DNA fragment B join at the homologous region a. The double-stranded DNA fragment B and the double-stranded DNA fragment C join at the homologous region b. This produces linear DNA in which the double-stranded DNA fragment A, the double-stranded DNA fragment B, and the double-stranded DNA fragment C are joined in this order. In this case, a homologous region c is further placed both at the downstream end of the double-stranded DNA fragment C and at the upstream end of the double-stranded DNA fragment A. As a result, the double-stranded DNA fragment A and the double-stranded DNA fragment B join at the homologous region a, and the double-stranded DNA fragment B and the double-stranded DNA fragment C join at the homologous region b, and the double-stranded DNA fragment C and the double-stranded DNA fragment A join at the homologous region c. This produces circular DNA in which the double-stranded DNA fragment A, the double-stranded DNA fragment B, and the double-stranded DNA fragment C are joined in this order.

The homologous region and the complementary region may be any sequence as long as the single strands having the region can specifically hybridize with each other in the reaction solution of the joining reaction. The base pair (bp) length, GC ratio, etc. of the region may be appropriately determined with reference to a general method for designing probes and primers. In general, the base pair length of the homologous region needs a certain length in order to suppress non-specific hybridization and accurately join the linear double-stranded DNA fragments of interest. On the other hand, when the base pair length of the homologous region is too long, the binding efficiency may decrease. In the present invention, the base pair length of the homologous region or complementary region is preferably 10 base pairs (bp) or more, more preferably 15 bp or more, and further preferably 20 bp or more. The base pair length of the homologous region or complementary region is preferably 500 bp or less, more preferably 300 bp or less, and further preferably 200 bp or less.

In the DNA production method according to the present invention, the length of the DNA fragments to be joined to each other is not particularly limited. For example, the length of the linear double-stranded DNA fragment is preferably 50 bp or more, more preferably 100 bp or more, and further preferably 200 bp or more. The length of the single-stranded DNA fragment is preferably 50 bases (base) or more, more preferably 100 bases or more, and further preferably 200 bases or more. In the DNA production method according to the present invention, it is possible to join double-stranded DNA fragments of 325 kbp. The length of the DNA fragment to be joined may vary depending on the type.

In the DNA production method according to the present invention, the whole or part of the homologous region of linear double-stranded DNA fragments to be joined to each other needs to be a double-stranded structure in which two single-stranded DNAs hybridize. That is, the linear double-stranded DNA fragment may be a complete linear double-stranded DNA fragment without gaps or nicks, and may be a linear double-stranded DNA fragment having a single-stranded structure at one or more positions. For example, the linear double-stranded DNA fragment to be joined may be a blunt end or a protruding end. By the DNA production method according to the present invention, it is possible to join a linear double-stranded DNA fragment with a blunt end and a linear double-stranded DNA fragment with a protruding end.

The molar ratio of each DNA fragment included in the reaction solution is preferably the same as the ratio of the number of molecules of each DNA fragment constituting the joined body of interest. By matching the number of DNA fragments in the reaction system at the start of the joining reaction, the joining reaction can be performed more efficiently. For example, when the DNA fragments to be joined are all different types, the molar concentration of each DNA fragment contained in the reaction solution is preferably the same.

The total amount of DNA fragments included in the reaction solution is not particularly limited. Since a sufficient amount of the joined product can be easily obtained, the total concentration of DNA fragments contained in the reaction solution at the start of the joining reaction is preferably 0.01 nM or more, more preferably 0.1 nM or more, and further preferably 0.3 nM or more. Since the joining efficiency is higher and suitable for joining multiple fragments, the total concentration of DNA fragments contained in the reaction solution at the start of the joining reaction is preferably 100 nM or less, more preferably 50 nM or less, further preferably 25 nM or less, and particularly preferably 20 nM or less.

In the DNA production method according to the present invention, the size of the joined body obtained by the joining reaction is not particularly limited. For example, the size of the obtained joined body is preferably 1000 bases or more, more preferably 5000 bases or more, further preferably 10000 bases or more, and further more preferably 20000 bases or more. The DNA production method according to the present invention makes it possible to obtain a joined body having a length of 300,000 bases or more, preferably 500,000 bases or more, more preferably 2,000,000 bases or more.

The exonuclease used in the present invention is an enzyme that sequentially hydrolyzes linear DNA from the 3'-end or 5'-end. The exonuclease used in the present invention is not particularly limited in its type or biological origin as long as it has enzymatic activity that sequentially hydrolyzes from the 3'-end or 5'-end of linear DNA. Examples of enzymes that sequentially hydrolyze from the 3'-end (3'→ 5' exonuclease) include linear double-stranded DNA-specific 3'→ 5' exonuclease such as exonuclease III family type-AP (apurinic / apyrimidinic) endonuclease and single-stranded DNA-specific 3'→ 5' exonuclease such as DnaQ superfamily protein. Examples of exonuclease III family type-AP endonucleases include exonuclease III (derived from Escherichia coli), ExoA (Bacillus subtilis homologue of exonuclease III), Mth212 (Archaea homologue of exonuclease III), and AP endonuclease I (human homologue of exonuclease III). Examples of DnaQ superfamily proteins include exonuclease I (derived from E. coli), exonuclease T (Exo T) (also known as RNase T), exonuclease X, DNA polymerase III epsilon subunit, DNA polymerase I, DNA polymerase II, T7 DNA polymerase, T4 DNA polymerase, Klenow DNA polymerase 5, Phi29 DNA polymerase, ribonuclease III (RNase D), and oligoribonuclease (ORN). Examples of enzymes that sequentially hydrolyze linear DNA from the 5'-end (5'→ 3' exonuclease) include λ exonuclease, exonuclease VIII, T5 exonuclease, T7 exonuclease, and RecJ exonuclease.

As the exonuclease used in the present invention, 3'→ 5' exonuclease is preferred, from the viewpoint of a good balance between the processivity of cleaving linear double-stranded DNA fragments and the joining efficiency in the presence of RecA family recombinase proteins. Among these, linear double-stranded DNA-specific 3'→ 5' exonuclease is more preferable, exonuclease III family type-AP endonuclease is further preferable, and exonuclease III is particularly preferable.

In the present invention, the reaction solution preferably contains both the linear double-stranded DNA-specific 3'→ 5' exonuclease and the single-stranded DNA-specific 3'→ 5' exonuclease as exonucleases. The joining efficiency is further improved by combining the single-stranded DNA-specific 3'→ 5' exonuclease with the linear double-stranded DNA-specific 3'→ 5' exonuclease, compared to the case of using only the linear double-stranded DNA-specific 3'→ 5' exonuclease. It is presumed that the 3'- protruding end formed secondary in a joined body by the linear double-stranded DNA-specific 3'→ 5'exonuclease and RecA is digested by the single-stranded DNA specific 3'→ 5'exonuclease, whereby the joining efficacy is improved. Since the joining efficiency can be particularly improved, the exonuclease contained in the reaction solution of the present invention is preferably a combination of an exonuclease III family type-AP endonuclease and one or more types of single-stranded DNA-specific 3'→ 5' exonucleases. A combination of an exonuclease III family type-AP endonuclease and one or more types of DnaQ superfamily proteins is more preferable. A combination of exonuclease III and exonuclease I, or a combination of exonuclease III, exonuclease I and exonuclease T is particularly preferred.

In the present invention, the concentration of exonuclease in the reaction solution of the joining reaction at the start of the joining reaction is preferably, for example, 1 to 1000 mU/µL, more preferably 5 to 1000 mU/µL, further preferably 5 to 500 mU/µL, and further more preferably 10 to 150 mU/µL. In particular, when the exonuclease is a linear double-stranded DNA-specific 3'→ 5' exonuclease, the concentration of the linear double-stranded DNA-specific 3'→ 5' exonuclease in the reaction solution at the start of the joining reaction is, for example, preferably 5 to 500 mU/µL, more preferably 5 to 250 mU/µL, further preferably 5 to 150 mU/µL, and further more preferably 10 to 150 mU/µL. When the exonuclease is a single-stranded DNA-specific 3'→ 5' exonuclease, the concentration of the single-stranded DNA-specific 3'→ 5' exonuclease in the reaction solution at the start of the joining reaction is, for example, preferably 1 to 10000 mU/µL, more preferably 100 to 5000 mU/µL, further preferably 200 to 2000 mU/µL. When a linear double-stranded DNA-specific 3'→ 5' exonuclease and a single-stranded DNA-specific 3'→ 5' exonuclease are used in combination, the concentration of each exonuclease in the reaction solution at the start of the joining reaction can be a preferred concentration of each of the above exonucleases.

In the present invention and the present specification, the RecA family recombinase protein means a protein having RecA family recombinase activity. This activity includes a function of polymerizing on single-stranded or double-stranded DNA to form a filament, hydrolysis activity for nucleoside triphosphates such as ATP (adenosine triphosphate), and a function of searching for a homologous region and performing homologous recombination. Examples of the RecA family recombinase proteins include Prokaryotic RecA homolog, bacteriophage RecA homolog, archaeal RecA homolog, eukaryotic RecA homolog, and the like. Examples of Prokaryotic RecA homologs include E. coli RecA; RecA derived from highly thermophilic bacteria such as Thermus bacteria such as Thermus thermophiles and Thermus aquaticus, Thermococcus bacteria, Pyrococcus bacteria, and Thermotoga bacteria; RecA derived from radiation-resistant bacteria such as Deinococcus radiodurans. Examples of bacteriophage RecA homologs include T4 phage UvsX. Examples of archaeal RecA homologs include RadA. Examples of eukaryotic RecA homologs include Rad51 and its paralog, and Dcm1. The amino acid sequences of these RecA homologs can be obtained from databases such as NCBI (http://www.ncbi.nlm.nih.gov/).

The RecA family recombinase protein used in the present invention may be a wild-type protein or a variant thereof. The variant is a protein in which one or more mutations that delete, add or replace 1 to 30 amino acids are introduced into a wild-type protein and which retains the RecA family recombinase activity. Examples of the variants include variants with amino acid substitution mutations that enhance the function of searching for homologous regions in wild-type proteins, variants with various tags added to the N-terminal or C-terminus of wild-type proteins, and variants with improved heat resistance (WO 2016/013592). As the tag, for example, tags widely used in the expression or purification of recombinant proteins such as His tag, HA (hemagglutinin) tag, Myc tag, and Flag tag can be used. The wild-type RecA family recombinase protein means a protein having the same amino acid sequence as that of the RecA family recombinase protein retained in organisms isolated from nature.

The RecA family recombinase protein used in the present invention is preferably a variant that retains the RecA family recombinase protein. Examples of the variants include a F203W mutant in which the 203^{rd} amino acid residue phenylalanine of E. coli RecA is substituted with tryptophan, and mutants in which phenylalanine corresponding to the 203^{rd} phenylalanine of E. coli RecA is substituted with tryptophan in various RecA homologs.

In the present invention, the amount of the RecA family recombinase protein in the reaction solution of the joining reaction is not particularly limited. In the present invention, the amount of the RecA family recombinase protein in the reaction solution of the joining reaction at the start of the joining reaction is preferably, for example, 0.01 to 100 µM, more preferably 0. 1 to 100 µM, further preferably 0.1 to 50 µM, further more preferably 0.5 to 10 µM, and particularly preferably 1.0 to 5.0 µM.

It is required that nucleoside triphosphates or deoxynucleotide triphosphates for RecA family recombinase proteins to exhibit RecA family recombinase activity. For this reason, the reaction solution of the joining reaction in the present invention contains at least one of nucleoside triphosphate and deoxynucleotide triphosphate. As the nucleoside triphosphate contained in the reaction solution for the joining reaction in the present invention, it is preferable to use one or more selected from the group consisting of dATP (deoxyadenosine triphosphate), dGTP (deoxyguanosine triphosphate), dCTP (deoxycytidine triphosphate), and dTTP (deoxythymidine triphosphate). It is particularly preferable to use dATP. The total amount of nucleoside triphosphate and deoxynucleotide triphosphate contained in the reaction solution is not particularly limited as long as it is sufficient for RecA family recombinase protein to exhibit RecA family recombinase activity. In the present invention, the nucleoside triphosphate concentration or deoxynucleotide triphosphate concentration of the reaction solution for performing the joining reaction at the start of the joining reaction is preferably, for example, 1 µM or more, more preferably 10 µM or more, and further preferably 30 µM or more. On the other hand, when the nucleoside triphosphate concentration in the reaction solution is too high, the joining efficiency of multiple fragments may decrease slightly. Therefore, the nucleoside triphosphate concentration or deoxynucleotide triphosphate concentration of the reaction solution at the start of the joining reaction is preferably 1000 µM or less, more preferably 500 µM or less, and further preferably 300 µM or less.

Magnesium ions (Mg²⁺) are required for RecA family recombinase protein to exhibit RecA family recombinase activity and for exonuclease to exhibit exonuclease activity. Therefore, the reaction solution of the joining reaction in the present invention contains a magnesium ion source. The magnesium ion source is a substance that provides magnesium ions in the reaction solution. Examples thereof include magnesium salts such as magnesium acetate [Mg (OAc) z], magnesium chloride [MgCl ₂], and magnesium sulfate [MgSO ₄]. A preferred magnesium ion source is magnesium acetate.

In the present invention, the concentration of the magnesium ion source in the reaction solution of the joining reaction is not particularly limited, as long as RecA family recombinase protein can exhibit RecA family recombinase activity and exonuclease can exhibit exonuclease activity. The magnesium ion source concentration of the reaction solution at the start of the joining reaction is, for example, preferably 0.5 mM or more, and more preferably 1 mM or more. On the other hand, when the magnesium ion concentration in the reaction solution is too high, the exonuclease activity becomes too strong, and the joining efficiency of multiple fragments may decrease. For this reason, the magnesium ion source concentration of the reaction solution at the start of the joining reaction is preferably, for example, 20 mM or less, more preferably 15 mM or less, further preferably 12 mM or less, and further more preferably 10 mM or less.

The reaction solution of the joining reaction in the present invention is prepared, for example, by adding DNA fragments, a RecA family recombinase protein, an exonuclease, at least one of nucleoside triphosphate and deoxynucleotide triphosphate and a magnesium ion source into a buffer. The buffer solution is not particularly limited as long as it is suitable for use at pH 7 to 9, preferably pH 8. Examples of the buffers include Tris-HCl, Tris-OAc, Hepes-KOH, phosphate buffer, MOPS-NaOH, and Tricine-HCl. A preferred buffer is Tris-HCl or Tris-OAc. The concentration of the buffer solution is not particularly limited and can be appropriately selected by those skilled in the art. In the case of Tris-HCl or Tris-OAc, for example, the concentration of the buffer solution can be selected 10 mM to 100 mM, preferably 10 mM to 50 mM, more preferably 20 mM.

In the present invention, when UvsX is used as the RecA family recombinase protein, it is preferable that the reaction solution of the joining reaction further contains T4 phage UvsY UvsY is a mediator of homologous recombination in T4 phage. In T4 phage, first, single-stranded DNA is bound to gp32 (single-stranded DNA binding protein) to form a single-stranded DNA-gp32 complex. Next, the single-stranded DNA binds to uvsX so that gp32 in the complex is replaced with uvsX, and then homologous recombination is caused. UvsY promotes the binding of single-stranded DNA and uvsX by destabilizing the interaction of single-stranded DNA-gp32 and stabilizing the interaction of single-stranded DNA-uvsX, and thus promotes homologous recombination reaction (Bleuit et al., Proceedings of the National Academy of Sciences of the United States of America, 2001, vol.98(15), p.8298-8305) . Also in the present invention, by using UvsY together with UvsX, the joining efficiency is further promoted.

The reaction solution of the joinig reaction in the present invention contains a DNA fragment, a RecA family recombinant enzyme protein, exonuclease, at least one of nucleoside triphosphate and deoxynucleotide triphosphate, and a magnesium ion source. The reaction solution preferably further contains a regenerating enzyme for nucleoside triphosphate or deoxynucleotide triphosphate and its substrate. By regenerating nucleoside triphosphates or deoxynucleotide triphosphates in the reaction solution, a large number of DNA fragments can be joined more efficiently. Examples of combinations of regenerating enzymes and their substrates to regenerate nucleoside triphosphate or deoxynucleotide triphosphate include combination of creatine kinase and creatine phosphate, combination of pyruvate kinase and phosphoenolpyruvate, combination of acetate kinase and acetyl phosphate, combination of polyphosphate kinase and polyphosphate, and combination of nucleoside diphosphate kinase and nucleoside triphosphate. The nucleoside triphosphate used as the substrate (phosphate supply source) of nucleoside diphosphate kinase may be any of ATP, GTP, CTP, and UTP. In addition, examples of the regenerating enzyme include myokinase.

In the present invention, the concentrations of regenerating enzyme for nucleoside triphosphate and its substrate in the reaction solution of the joining reaction are not particularly limited, as long as the concentration is sufficient to enable regeneration of the nucleoside triphosphate during the joining reaction in the reaction solution. For example, when using creatine kinase and creatine phosphate, the concentration of creatine kinase in the reaction solution of the joining reaction in the present invention is preferably 1 to 1000 ng/µL, more preferably 5 to 1000 ng/µL, further preferably 5 to 500 ng/µL, and further more preferably 5 to 250 ng/µL. The concentration of creatine phosphate in the solution is preferably 0.4 to 20 mM, more preferably 0.4 to 10 mM, and further preferably 1 to 7 mM.

When multiple fragments are joined in the desired order, the base sequence of the homologous region or complementary region is preferably different for each combination of DNA fragments to be joined. However, under the same temperature condition, a single-stranded homologous region having a high content rate of G (guanine base) and C (cytosine base) tends to form a secondary structure. On the other hand, a homologous region having a high content rate of A (adenine base) and T (thymine base) has low hybridization efficiency. The joining efficiency may thus be lowered. By suppressing the secondary structure formation of single-stranded DNA and promoting specific hybridization, the joining of DNA fragments can be promoted.

Therefore, it is preferable to add a substance that suppresses the formation of secondary structure of single-stranded DNA and promotes specific hybridization into the reaction solution of the joining reaction in the present invention. Examples of the substances include dimethyl sulfoxide (DMSO) and tetramethylammonium chloride (TMAC). DMSO suppresses secondary structure formation of GC-rich base pairs. TMAC promotes specific hybridization. In the present invention, when the substance that suppresses the formation of secondary structure of single-stranded DNA and promotes specific hybridization is added into the reaction solution of the joining reaction, the concentration of the substance is not particularly limited, as long as the effect of promoting DNA fragment joining by the substance is obtained. For example, when DMSO is used as the substance, the concentration of DMSO in the reaction solution of the joining reaction in the present invention is preferably 5-30% by volume, more preferably 8-25% by volume, and further preferably 8-20% by volume. When TMAC is used as the substance, the concentration of TMAC in the reaction solution of the joining reaction in the present invention is preferably 60 to 300 mM, more preferably 100 to 250 mM, and further preferably 100 to 200 mM.

In the present invention, it is preferable to add a substance having a macromolecular crowding effect to the reaction solution of the joining reaction. The macromolecular crowding effect can enhance the interaction between DNA molecules and promote the joining of DNA fragments. Examples of substances include polyethylene glycol (PEG) 200-20000, polyvinyl alcohol (PVA) 200-20000, dextran 40-70, Ficoll 70, and bovine serum albumin (BSA). In the present invention, when the substance having a macromolecular crowding effect is added into the reaction solution of the joining reaction, the concentration of the substance is not particularly limited as long as the substance can promote the joining of DNA fragments. For example, when using PEG 8000 as the substance, the concentration of the substance in the reaction solution of the joining reaction is preferably 2 to 20% by mass, more preferably 2 to 10% by mass, and further preferably 4 to 6% by mass.

In the present invention, the reaction solution of the joining reaction may further contain an alkali metal ion source. The alkali metal ion source is a substance that provides alkali metal ions in the reaction solution. In the present invention, the alkali metal ion contained in the reaction solution of the joining reaction is preferably sodium ion (Na ⁺) or potassium ion (K ⁺). Examples of the alkali metal ion source include potassium glutamate [KGlu], potassium aspartate, potassium chloride, potassium acetate [KOAc], sodium glutamate, sodium aspartate, sodium chloride, and sodium acetate. In the present invention, the alkali metal ion source contained in the reaction solution of the joining reaction is preferably potassium glutamate or potassium acetate. Potassium glutamate is particularly preferred because the joining efficiency of multiple fragments is improved. The concentration of the alkali metal ion source in the reaction solution at the start of the joining reaction is not particularly limited. For example, the concentration of the alkali metal ion source is preferably a concentration that can be adjusted to the concentration of alkali metal ions in the reaction solution to be 10 mM or more, preferably in the range of 30 to 300 mM, more preferably in the range of 50 to 150 mM.

In the present invention, the reaction solution of the joining reaction may further contain a reducing agent. Examples of the reducing agent include dithiothreitol (DTT), β-mercaptoethanol (2-mercaptoethanol), tris (2-carboxyethyl) phosphine (TCEP), and glutathione. A preferred reducing agent is DTT. The reducing agent may be contained in the reaction solution at 1.0 to 15.0 mM, preferably 2.0 to 10.0 mM.

In the DNA production method according to the present invention, the joining reaction is performed by incubating the reaction solution for a predetermined time under an isothermal condition at a temperature at which the RecA family recombinant enzyme protein and exonuclease in the reaction solution can exert their enzyme activities. The reaction solution is prepared by containing two or more kinds of DNA fragments, a RecA family recombinant enzyme protein, a nucleoside triphosphate, and a magnesium ion source, and further, if necessary, exonuclease, a set of nucleoside triphosphate-regenerating enzyme and its substrate, a substance that suppresses secondary structure formation of single-stranded DNA and promotes specific hybridization, a substance having a macromolecular crowding effect, an alkali metal ion source and a reducing agent into a buffer solution. The reaction temperature for the joining reaction is preferably within a temperature range of 25 to 48 °C, and more preferably within a temperature range of 27 to 45 °C. In particular, when the length of the homologous region or complementary region is 50 bases or more, the reaction temperature of the joining reaction is preferably within the temperature range of 30 to 45 °C, more preferably within the temperature range of 37 to 45 °C, and further preferably within a temperature range of 40 to 43 °C. On the other hand, when the length of the homologous region or complementary region is 50 bases or less, the reaction temperature of the joining reaction is preferably within the temperature range of 27 to 43 °C, more preferably within the temperature range of 27 to 37 °C, and further preferably within the temperature range of 27 to 33 °C. In the present specification, "isothermal condition" means that the temperature is maintained within the range of ± 3 °C or ± 1 °C during the reaction. The reaction time of the joining reaction is not particularly limited, and can be, for example, 15 minutes to 6 hours, preferably 15 minutes to 2 hours.

As shown in FIG. 1, there are gaps and nicks in the joined body (linear or circular DNA) obtained by the joining reaction. A gap is a state in which one or more consecutive nucleotides are missing in double-stranded DNA. A nick is a state in which a phosphodiester bond between adjacent nucleotides in a double-stranded DNA is cleaved. In the DNA production method according to the present invention, it is preferable to repair gaps and nicks in the obtained joined body using gap repair enzymes and dNTP, after the joining reaction. By repairing gaps and nicks, the joined body can be made into complete double-stranded DNA.

Specifically, gaps and nicks of the joined body are repaired by adding gap repair enzymes and dNTP into the reaction solution after the joining reaction and incubating for a predetermined time under isothermal conditions at which the gap repair enzyme group can exert enzyme activity. The type of gap repair enzymes are not particularly limited and biological origins as long as they have the ability to repair gaps and nicks in double-stranded DNA. As the gap repair enzymes, for example, an enzyme having DNA polymerase activity and an enzyme having DNA ligase activity can be used in combination. When using DNA ligase derived from Escherichia coli as the DNA ligase, NAD (nicotinamide adenine dinucleotide), which is its cofactor, is contained in the range of 0.01 to 1.0 mM in the reaction solution. The treatment with the gap repair enzymes may be performed, for example, at 25 to 40 °C for 5 to 120 minutes, preferably 10 to 60 minutes.

dNTP is a general term for dATP, dGTP, dCTP, and dTTP. The concentration of dNTP contained in the reaction solution at the start of the repairing reaction may be, for example, in the range of 0.01 to 1 mM, and preferably in the range of 0.05 to 1 mM.

It is also preferable to further amplify the joined body (linear or circular DNA) with gaps and nicks repaired. A method for amplifying a joined body with gaps and nicks repaired is not particularly limited. The amplification can be performed by a general method for amplification using linear or circular DNA as a template.

In the DNA production method according to the present invention, when the joined body obtained by performing the gap and nick repairing reaction after the joining reaction is linaer, the joined body is preferably amplified by polymerase chain reaction (PCR). PCR can be performed by a conventional method.

In the DNA production method according to the present invention, when the joined body obtained by performing the gap and nick repairing reaction after the joining reaction is circular, the joined body is preferably amplified by a rolling circle amplification method (RCA). RCA can be performed by a conventional method.

In the DNA production method according to the present invention, when the joined body obtained by performing the gap and nick repairing reaction after the joining reaction is circular and has a replication origin sequence (origin of chromosome (oriC)) which is capable of binding to an enzyme having DnaA activity, the joined body is preferably amplified by a replication cycle reaction (RCR) amplification method. By performing the RCR amplification using the joined body obtained from the joining reaction directly as a template, that is, without gap and nick repair reactions, it is possible to obtain a circular joined body of complete double-stranded DNA without gaps and nicks as an amplification product.

For example, a known replication origin sequence existing in bacteria such as Escherichia coli and Bacillus subtilis can be obtained from a public database such as NCBI. It is also possible to obtain a replication origin sequence by cloning a DNA fragment that can bind to an enzyme having DnaA activity and analyzing the base sequence thereof.

Specifically, the RCR amplification method can be performed by forming a reaction mixture and incubating the formed reaction mixture. The reaction mixture includes the circular joined body obtained by the joining reaction as a template, a first enzyme group that catalyzes replication of circular DNA, a second enzyme group that catalyzes an Okazaki fragment joining reaction and synthesizes two sister circular DNAs constituting a catenane, a third enzyme group that catalyzes the separation of two sister circular DNAs, and dNTP. The two sister circular DNAs constituting a catenane are the two circular DNAs synthesized by DNA replication and in a connected state.

An example of a first enzyme group that catalyzes replication of circular DNA is an enzyme group set forth in Kaguni JM & Kornberg A. Cell. 1984, 38:183-90. Specifically, examples of the first enzyme group include one or more enzymes or enzyme groups selected from the group consisting of an enzyme having DnaA activity, one or more types of nucleoid protein, an enzyme or enzyme group having DNA gyrase activity, single-stranded binding protein (SSB), an enzyme having DnaB-type helicase activity, an enzyme having DNA helicase loader activity, an enzyme having DNA primase activity, an enzyme having DNA clamp activity, and an enzyme or enzyme group having DNA polymerase III* activity, and a combinations of all of the aforementioned enzymes or enzyme groups.

The enzyme having DnaA activity is not particularly limited in biological origin as long as it has initiator activity that is similar to that of DnaA, which is an initiator protein of Escherichia coli, and DnaA derived from Escherichia coli may be preferably used. The Escherichia coli-derived DnaA may be contained as a monomer in the reaction solution in an amount of 1 nM to 10 mM, preferably in an amount of 1 nM to 5 mM, 1 nM to 3 mM, 1 nM to 1.5 mM, 1 nM to 1.0 mM, 1 nM to 500 nM, 50 nM to 200 nM, or 50 nM to 150 nM, but without being limited thereby.

A nucleoid protein is protein in the nucleoid. The one or more types of nucleoid protein used in the present invention are not particularly limited in biological origin as long as it has an activity that is similar to that of the nucleoid protein of Escherichia coli. For example, Escherichia coli-derived IHF, namely, a complex of IhfA and/or IhfB (a heterodimer or a homodimer), or Escherichia coli-derived HU, namely, a complex of hupA and hupB can be preferably used. The Escherichia coli-derived IHF may be contained as a hetero/homo dimer in a reaction solution in a concentration range of 5 nM to 400 nM. Preferably, the Escherichia coli-derived IHF may be contained in a reaction solution in a concentration range of 5 nM to 200 nM, 5 nM to 100 nM, 5 nM to 50 nM, 10 nM to 50 nM, 10 nM to 40 nM, or 10 nM to 30 nM, but the concentration range is not limited thereto. The Escherichia coli-derived HU may be contained in a reaction solution in a concentration range of 1 nM to 50 nM, and preferably, may be contained therein in a concentration range of 5 nM to 50 nM or 5 nM to 25 nM, but the concentration range is not limited thereto.

An enzyme or enzyme group having DNA gyrase activity is not particularly limited in biological origin as long as it has activity that is similar to that of the DNA gyrase of Escherichia coli. For example, a complex of Escherichia coli-derived GyrA and GyrB can be preferably used. Such a complex of Escherichia coli-derived GyrA and GyrB may be contained as a heterotetramer in a reaction solution in a concentration range of 20 nM to 500 nM, and preferably, may be contained therein in a concentration range of 20 nM to 400 nM, 20 nM to 300 nM, 20 nM to 200 nM, 50 nM to 200 nM, or 100 nM to 200 nM, but the concentration range is not limited thereto.

A single-stranded binding protein (SSB) is not particularly limited in biological origin as long as it has activity that is similar to that of the single-stranded binding protein of Escherichia coli. For example, Escherichia coli-derived SSB can be preferably used. Such Escherichia coli-derived SSB may be contained as a homotetramer in a reaction solution in a concentration range of 20 nM to 1000 nM, and preferably, may be contained therein in a concentration range of 20 nM to 500 nM, 20 nM to 300 nM, 20 nM to 200 nM, 50 nM to 500 nM, 50 nM to 400 nM, 50 nM to 300 nM, 50 nM to 200 nM, 50 nM to 150 nM, 100 nM to 500 nM, or 100 nM to 400 nM, but the concentration range is not limited thereto.

An enzyme having DnaB-type helicase activity is not particularly limited in biological origin as long as it has activity that is similar to that of the DnaB of Escherichia coli. For example, Escherichia coli-derived DnaB can be preferablyused. Such Escherichia coli-derived DnaB may be contained as a homohexamer in a reaction solution in a concentration range of 5 nM to 200 nM, and preferably, may be contained therein in a concentration range of 5 nM to 100 nM, 5 nM to 50 nM, or 5 nM to 30 nM, but the concentration range is not limited thereto.

An enzyme having DNA helicase loader activity is not particularly limited in biological origin as long as it has activity that is similar to that of the DnaC of Escherichia coli. For example, Escherichia coli-derived DnaC can be preferably used. Such Escherichia coli-derived DnaC may be contained as a homohexamer in a reaction solution in a concentration range of 5 nM to 200 nM, and preferably, may be contained therein in a concentration range of 5 nM to 100 nM, 5 nM to 50 nM, or 5 nM to 30 nM, but the concentration range is not limited thereto.

An enzyme having DNA primase activity is not particularly limited in its biological origin as long as it has an activity that is similar to that of the DnaG of Escherichia coli. For example, Escherichia coli-derived DnaG can be preferably used. Such Escherichia coli-derived DnaG may be contained as a monomer in a reaction solution in a concentration range of 20nM to 1000 nM, and preferably, may be contained therein in a concentration range of 20nM to 800 nM, 50 nM to 800 nM, 100 nM to 800 nM, 200 nM to 800 nM, 250 nM to 800 nM, 250 nM to 500 nM, or 300 nM to 500 nM, but the concentration range is not limited thereto.

An enzyme having DNA clamp activity is not particularly limited in biological origin as long as it has activity that is similar to that of the DnaN of Escherichia coli. For example, Escherichia coli-derived DnaN can be preferably used. Such Escherichia coli-derived DnaN may be contained as a homodimer in a reaction solution in a concentration range of 10 nM to 1000 nM, and preferably, may be contained therein in a concentration range of 10 nM to 800 nM, 10 nM to 500 nM, 20 nM to 500 nM, 20 nM to 200 nM, 30 nM to 200 nM, or 30 nM to 100 nM, but the concentration range is not limited thereto.

An enzyme or enzyme group having DNA polymerase III* activity is not particularly limited in biological origin as long as it is an enzyme or enzyme group having activity that is similar to that of the DNA polymerase III* complex of Escherichia coli. For example, an enzyme group comprising any of Escherichia coli-derived DnaX, HolA, HolB, HolC, HolD, DnaE, DnaQ, and HolE, preferably, an enzyme group comprising a complex of Escherichia coli-derived DnaX, HolA, HolB, and DnaE, and more preferably, an enzyme comprising a complex of Escherichia coli-derived DnaX, HolA, HolB, HolC, HolD, DnaE, DnaQ, and HolE, can be preferably used. Such an Escherichia coli-derived DNA polymerase III* complex may be contained as a heteromultimer in a reaction solution in a concentration range of 2 nM to 50 nM, and preferably, may be contained therein in a concentration range of 2 nM to 40 nM, 2 nM to 30 nM, 2 nM to 20 nM, 5 nM to 40 nM, 5 nM to 30 nM, or 5 nM to 20 nM, but the concentration range is not limited thereto.

Examples of second enzyme groups that catalyze an Okazaki fragment maturation and synthesize two sister circular DNAs constituting a catenane may include, for example, one or more enzymes selected from the group consisting of an enzyme having DNA polymerase I activity, an enzyme having DNA ligase activity, and an enzyme having RNaseH activity, or a combination of these enzymes.

An enzyme having DNA polymerase I activity is not particularly limited in biological origin as long as it has activity that is similar to DNA polymerase I of Escherichia coli. For example, Escherichia coli-derived DNA polymerase I can be preferably used. Such Escherichia coli-derived DNA polymerase I may be contained as a monomer in a reaction solution in a concentration range of 10 nM to 200 nM, and preferably, may be contained therein in a concentration range of 20 nM to 200 nM, 20 nM to 150 nM, 20 nM to 100 nM, 40 nM to 150 nM, 40 nM to 100 nM, or 40 nM to 80 nM, but the concentration range is not limited thereto.

An enzyme having DNA ligase activity is not particularly limited in biological origin as long as it has activity that is similar to DNA ligase of Escherichia coli. For example, Escherichia coli-derived DNA ligase or the DNA ligase of T4 phage can be preferably used. Such Escherichia coli-derived DNA ligase may be contained as a monomer in a reaction solution in a concentration range of 10 nM to 200 nM, and preferably, may be contained therein in a concentration range of 15 nM to 200 nM, 20 nM to 200 nM, 20 nM to 150 nM, 20 nM to 100 nM, or 20 nM to 80 nM, but the concentration range is not limited thereto.

The enzyme having RNaseH activity is not particularly limited in terms of biological origin, as long as it has the activity of decomposing the RNA chain of an RNA-DNA hybrid. For example, Escherichia coli-derived RNaseH can be preferably used. Such Escherichia coli-derived RNaseH may be contained as a monomer in a reaction solution in a concentration range of 0.2 nM to 200 nM, and preferably, may be contained therein in a concentration range of 0.2 nM to 200 nM, 0.2 nM to 100 nM, 0.2 nM to 50 nM, 1 nM to 200 nM, 1 nM to 100 nM, 1 nM to 50 nM, or 10 nM to 50 nM, but the concentration range is not limited thereto.

An example of a third enzyme group that catalyzes the separation of two sister circular DNAs is an enzyme group set forth in, for example, the enzyme group described in Peng H & Marians KJ. PNAS. 1993, 90: 8571-8575. Specifically, examples of the third enzyme group include one or more enzymes selected from the group consisting of an enzyme having topoisomerase IV activity, an enzyme having topoisomerase III activity, and an enzyme having RecQ-type helicase activity; or a combination of the aforementioned enzymes.

The enzyme having topoisomerase III activity is not particularly limited in terms of biological origin, as long as it has the same activity as that of the topoisomerase III of Escherichia coli. For example, Escherichia coli-derived topoisomerase III can be preferably used. Such Escherichia coli-derived topoisomerase III may be contained as a monomer in a reaction solution in a concentration range of 20 nM to 500 nM, and preferably, may be contained therein in a concentration range of 20 nM to 400 nM, 20 nM to 300 nM, 20 nM to 200 nM, 20 nM to 100 nM, or 30 to 80 nM, but the concentration range is not limited thereto.

The enzyme having RecQ-type helicase activity is not particularly limited in terms of biological origin, as long as it has the same activity as that of the RecQ of Escherichia coli. For example, Escherichia coli-derived RecQ can be preferably used. Such Escherichia coli-derived RecQ may be contained as a monomer in a reaction solution in a concentration range of 20 nM to 500 nM, and preferably, may be contained therein in a concentration range of 20 nM to 400 nM, 20 nM to 300 nM, 20 nM to 200 nM, 20 nM to 100 nM, or 30 to 80 nM, but the concentration range is not limited thereto.

An enzyme having topoisomerase IV activity is not particularly limited in biological origin as long as it has activity that is similar to topoisomerase IV of Escherichia coli. For example, Escherichia coli-derived topoisomerase IV that is a complex of ParC and ParE can be preferably used. Such Escherichia coli-derived topoisomerase IV may be contained as a heterotetramer in a reaction solution in a concentration range of 0.1 nM to 50 nM, and preferably, may be contained therein in a concentration range of 0.1 nM to 40 nM, 0.1 nM to 30 nM, 0.1 nM to 20 nM, 1 nM to 40 nM, 1 nM to 30 nM, 1 nM to 20 nM, 1 nM to 10 nM, or 1 nM to 5 nM, but the concentration range is not limited thereto.

The first, second and third enzyme groups given above may be those that are commercially available, or they may be extracted from microorganisms and purified as necessary. Extraction and purification of enzymes from microorganisms may be performed as necessary using means that are available to a person skilled in the art.

When enzymes other than the above described Escherichia coli-derived enzymes are used as the first, second and third enzyme groups, they may be each used in a concentration range corresponding, as an enzyme activity unit, to the concentration range that is specified with respect to the above described Escherichia coli-derived enzyme.

As the dNTP contained in the reaction mixture in the RCR amplification method, the same dNTPs as those used in the DNA production method according to the present invention can be used.

If necessary, the reaction mixture prepared in the RCR amplification method may further contains a magnesium ion source, an alkali metal ion source, and ATP.

In the RCR amplification method, the concentration of ATP contained in the reaction mixture at the start of the reaction may be, for example, in the range of 0.1 to 3 mM, preferably in the range of 0.1 to 2 mM, more preferably in the range of 0.1 to 1.5 mM, further preferably in the range of 5 to 1.5 mM.

As the magnesium ion source to be included in the reaction mixture in the RCR amplification method, the same materials as those used in the DNA production method according to the present invention can be used. In the RCR amplification method, the concentration of the magnesium ion source contained in the reaction mixture at the start of the reaction may be, for example, a concentration that is necessary for providing 5 to 50 mM magnesium ions into the reaction mixture.

As the alkali metal ion source to be included in the reaction mixture in the RCR amplification method, the same materials as those used in the DNA production method according to the present invention can be used. In the RCR amplification method, the concentration of the alkali metal ion source contained in the reaction mixture at the start of the reaction may be, for example, a concentration that is necessary for providing alkali metal ions in a range of 100 mM or more, and preferably 100 mM to 300 mM, into the reaction solution, but the concentration is not limited thereto.

In the RCR amplification method, the amount of the joined body to be contained in the reaction mixture is not particularly limited. For example, at the start of the reaction, the joined body may be present at a concentration of 10ng/µL or less, 5ng/µL or less, 1ng/µL or less, 0.8ng/µL or less, 0.5ng/µL or less, or 0.3ng/µL or less in the mixture.

By incubating the prepared reaction mixture under isothermal conditions at a predetermined temperature, only circular DNA containing a replication origin sequence capable of binding to an enzyme having DnaA activity is amplified. The reaction temperature in the RCR amplification is not particularly limited as long as the DNA replication reaction can proceed. The temperature may be, for example, more specifically in the range of 20 to 80 °C, 25 to 50 °C, or 25 to 40 °C, which is the optimal temperature of the DNA polymerase. The reaction time in RCR amplification can be appropriately set according to the amount of the amplification product of the target circular joined body. The reaction time may be, for example, 30 minutes to 24 hours.

The RCR amplification can also be performed by incubating the prepared reaction mixture according to a temperature cycle that repeats incubation at 30 °C or higher and incubation at 27 °C or lower. The incubation at 30 °C or higher is not particularly limited, as long as the temperature is in a temperature range capable of initiating the replication of circular DNAincluing oriC. For example, the temperature may be 30 to 80 °C, 30 to 50 °C, 30 to 40 °C, or 37 °C. The incubation at 30 °C or higher may be performed for 10 seconds to 10 minutes per cycle, although it is not particularly limited thereto. The incubation at 27 °C or lower is not particularly limited, as long as it is a temperature, at which initiation of replication is suppressed and the elongation reaction of DNA progresses. For example, the temperature may be 10 to 27 °C, 16 to 25 °C, or 24 °C. The incubation at 27 °C or lower may be preferably determined depending on the length of circular DNA to be amplified, but is not particularly limited thereto. For example, the incubation may be performed for 1 to 10 seconds per 1000 bases in a single cycle. The number of temperature cycles is not particularly limited, but may be 10 to 50 cycles, 20 to 40 cycles, 25 to 35 cycles, or 30 cycles.

Before being used for the gap and nick-repairing reaction and for the RCR amplification as a template, the joined body obtained by the joining reaction is preferably subjected to heat treatment incubation at 50 to 70 °C, followed by rapid cooling. The treatment time of the heat treatment is not particularly limited, and may be, for example, 1 to 15 minutes, preferably 2 to 10 minutes. The temperature of the rapid cooling is not particularly limited, and for example, 10 °C or lower, preferably 4 °C or lower. The cooling rate is preferably 50°C/min or more, more preferably 70°C/min or more, and further preferably 85°C/min or more. For example, the reaction mixture in the container can be rapidly cooled after the heat treatment by leaving it directly on ice or contacting it with a metal block adjusted to 4 °C or lower.

The reaction solution immediately after the joining reaction contains joined bodies obtained by non-specific joining. Non-specific joining can be dissociated by the heat treatment and rapid cooling of the reaction solution. For this reason, gap- and nick-repairing reactions and RCR amplification reactions are performed using the joined body after the heat treatment and rapid cooling as a template. Thereby, non-specific joining is suppressed, and a complete double-stranded DNA of the joined body of interest can be obtained efficiently.

In the DNA production method according to the present invention, the amplification of the linear or circular joined body obtained by the joining reaction is carried out in the microorganism by introducing the joined body to the microorganism. This can be performed using an enzyme or the like of the microorganism. The joined body to be introduced into the microorganism may be a joined body before the gap and nick-repairing reaction, or a joined body after the repairing reaction. Even when a joined body having a gap and nick is directly introduced into a microorganism, a joined body in a complete double-stranded DNA state without a gap and a nick can be obtained as an amplification product. Examples of the microorganism into which the joined body is introduced include Escherichia coli, Bacillus subtilis, actinomycetes, archaea, yeast, filamentous fungi and the like. Introduction of the joined body into the microorganism can be performed by a conventional method such as an electroporation method. The amplified joined body can be collected from the microorganism by a conventional method.

### [DNA fragment-joining kit]

The DNA fragment-joining kit according to the present invention is a kit for joining two or more types of DNA fragments to each other at regions where the base sequences are homologous to obtain linear or circular DNA, and contains the RecA family recombinase protein. When used for joining linear double-stranded DNA fragments, the kit preferably further contains an exonuclease. The RecA family recombinase protein and the exonuclease provided in the kit are added to a solution containing two or more types of DNA fragments to be joined. Thus, the DNA production method according to the present invention can be performed more easily, and the joined body of interest can be easily obtained. As the RecA family recombinase protein contained in the kit, the same enzymes as those used in the DNA production method according to the present invention can be used. The exonuclease contained in the kit is preferably 3'→ 5' exonuclease. More preferably, the kit contains at least a linear double-stranded DNA-specific 3'→ 5' exonuclease, and further preferably, both a linear double-stranded DNA-specific 3'→ 5' exonuclease and a single-stranded DNA specific 3'→ 5' exonuclease.

The DNA fragment-joining kit according to the present invention preferably further includes a regenerating enzyme for nucleoside triphosphate or deoxynucleotide triphosphate, and its substrate. The DNA fragment-joining kit according to the present invention may include one or more selected from the group consisting of nucleoside triphosphate, deoxynucleotide triphosphate, a magnesium ion source, an alkali metal ion source, dimethyl sulfoxide, tetramethylammonium chloride, polyethylene glycol, dithiothreitol, and buffer. Any of those used in the DNA production method according to the present invention can be used as they are.

The DNA fragment-joining kit according to the present invention preferably further includes a document describing a protocol for performing the DNA production method according to the present invention using the kit. The protocol may be described on the surface of the container containing the kit.

### [Examples]

Next, the present invention will be described in more detail by showing examples, but the invention is not limited to the following examples.

### [Example 1]

Two or more types of linear double-stranded DNA fragments were joined using RecA family recombinase protein and 3'→ 5' exonuclease. In the reaction, the effects of magnesium ion source concentration and ATP concentration in the reaction solution were investigated.

DCW1 to DCW7 (SEQ ID NO: 1 to SEQ ID NO: 7) which were 591bp linear double-stranded DNA fragments were used as the linear double-stranded DNA fragments to be joined. The region from the end to 60 bases of each linear double-stranded DNA fragment was a homologous region. That is, the 60 bases region from 532^{rd} to 591^{st} in DCW1 was the homologous region for joining to DCW2, and consisted of the same base sequence as 60 bases from 1^{st} to 60^{th} in DCW2. The 60 bases region from 532^{rd} to 591^{st} in DCW2 was the homologous region for joining to DCW3, and consisted of the same base sequence as 60 bases from 1^{st} to 60^{th} in DCW3. The 60 bases region from 532^{rd} to 591^{rd} in DCW3 was the homologous region for joining to DCW4, and consisted of the same base sequence as 60 bases from 1^{st} to 60^{th} in DCW4. The 60 bases region from 532^{rd} to 591^{st} in DCW4 was the homologous region for joining to DCW5, and consisted of the same base sequence as 60 bases from 1^{st} to 60^{th} in DCW5. The 60 bases region from 532^{rd} to 591^{st} in DCW5 was the homologous region for joining to DCW6, and consisted of the same base sequence as 60 bases from 1^{st} to 60^{th} in DCW6. The 60 bases region from 532^{rd} to 591^{st} in DCW6 was the homologous region for joining to DCW7, and consisted of the same base sequence as 60 bases from 1^{st} to 60^{th} in DCW7.

The wild-type of E. coli RecA (SEQ ID NO: 61) was used as the RecA family recombinase protein, and exonuclease III was used as the 3'→ 5' exonuclease.

Specifically, first, the reaction solutions consisting of 1nM each of DCW1 to DCW7, 1 µM of RecA, 40mU/µL of exonuclease III, 20 mM of Tris-HCl (pH8.0), 4 mM of DTT, 1 mM or 10 mM of magnesium acetate, 30 µM, 100 µM, 300 µM, or 1000 µM of ATP, 4mM of creatine phosphate, 20ng/µL of creatine kinase, 50 mM of potassium glutamate, 150 mM of TMAC, 5% by mass of PEG8000, 10% by volume of DMSO were prepared. Next, these reaction solutions were incubated at 42 °C for 2 hours to perform the joining reaction. 1 µL of the reaction solution after the reaction was subjected to agarose gel electrophoresis, and the separated bands were stained with SYBR (registered trademark) Green.

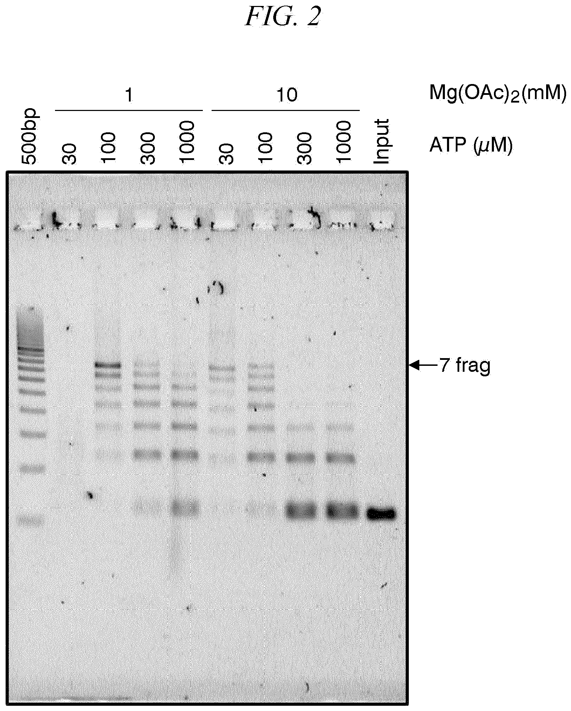

The staining results are shown in FIG. 2. In the figure, "500 bp" indicates the lane in which a DNA ladder marker consisting of 500 bp to 5 kbp bands (total of 10) at intervals of 500 bp was run, and "Input" indicates the lane in which 1 µL of the solution containing 1 nM each of DCW1 to DCW7 was run. In the figure, "7 frag" indicates a band of a joined body in which all seven fragments of DCW1 to DCW7 were joined.

Among the reaction solutions with 1 mM of magnesium acetate, almost no joined body was detected in the reaction solution with 30 µM of ATP, but in the reaction solutions with 100 µM, 300 µM, and 1000 µM of ATP, bands of 6 types of joined bodies, which were from 2-fragment joined body to 7-fragment joined body, were detected. Comparing the results of the reaction solutions with 100 µM, 300 µM, and 1000 µM of ATP, the reaction solution with 100 µM of ATP contained the largest amount of the joined body obtained by joining all 7 types of fragments, and bands of unjoined fragments were not detected. In contrast, in the reaction solution with 1000 µM of ATP, the band of the 7-fragment joined body was very thin, and many unjoined fragments remained.

On the other hand, among the reaction solutions with 10 mM of magnesium acetate, bands of 6 types of joined bodies, from 2-fragment joined body to 7-fragment joined body, were detected in the reaction solutions with 30 µM and 100 µM of ATP. In the reaction solutions with 300 µM and 1000 µM of ATP, bands of joined bodies from 2-fragment joined body to 4-fragment joined body were detected, but bands of joined bodies with 5 or more fragments joined were not detected and many unjoined fragments remained.

Among all the samples, the production amount of the 7-fragment joined body was the highest in the reaction solution with 1 mM of magnesium acetate and 100 µM of ATP. These results suggested the following: In order to join multiple fragments, it is important to balance the magnesium ion concentration and ATP concentration in the reaction solution. And when the ATP concentration is too high, the joining reaction may be inhibited.

### [Example 2]

Two or more types of linear double-stranded DNA fragments were joined using RecA family recombinase protein and 3'→ 5' exonuclease. In the reaction, the effects of PEG 8000 concentration and ATP-regenerating system in the reaction solution were investigated.

Lter1 to Lter5 (SEQ ID NO: 54 to SEQ ID NO: 58) which are 3100 bp or 2650 bp linear double-stranded DNA fragments were used as the linear double-stranded DNA fragments to be joined. The region from the end to 60 bases of each linear double-stranded DNA fragment was a homologous region. That is, the 60 bases region from 3041^{st} to 3100^{th} in Lter1 was the homologous region for joining to Lter2, and consisted of the same base sequence as 60 bases from 1^{st} to 60^{th} in Lter2. The 60 bases region from 3041^{st} to 3100^{th} in Lter2 was the homologous region for joining to Lter3, and consisted of the same base sequence as 60 bases from 1^{st} to 60^{th} in Lter3. The 60 bases region from 3041^{st} to 3100^{th} in Lter3 was the homologous region for joining to Lter4, and consisted of the same base sequence as 60 bases from 1^{st} to 60^{th} in Lter4. The 60 bases region from 3041^{st} to 3100^{th} in Lter4 was the homologous region for joining to Lter5, and consisted of the same base sequence as 60 bases from 1^{st} to 60^{th} in Lter5.

The F203W mutant of E. coli RecA was used as the RecA family recombinase protein, and exonuclease III was used as the 3'→ 5' exonuclease. Creatine kinase was used as the ATP-regenerating enzyme, and creatine phosphate was used as the substrate.

Specifically, first, the reaction solutions consisting of 0.3 nM each of Lter1 to Lter5, 1 µM of the F203W mutant of RecA, 40 mU/µL of exonuclease III, 20 mM of Tris-HCl (pH 8.0), 10 mM of magnesium acetate, 100 µM of ATP, 4 mM of creatine phosphate, 20 ng/µL of creatine kinase, 150 mM potassium acetate, and 0 %, 2 %, 5 %, or 10 % by weight of PEG 8000 were prepared. Separately, the reaction solutions were prepared in the same manner except that they did not contain creatine phosphate and creatine kinase. Next, these reaction solutions were incubated at 30 °C for 30 minutes to perform the joining reaction. 4 µL of the reaction solution after the reaction was subjected to agarose gel electrophoresis, and the separated bands were stained with SYBR Green.

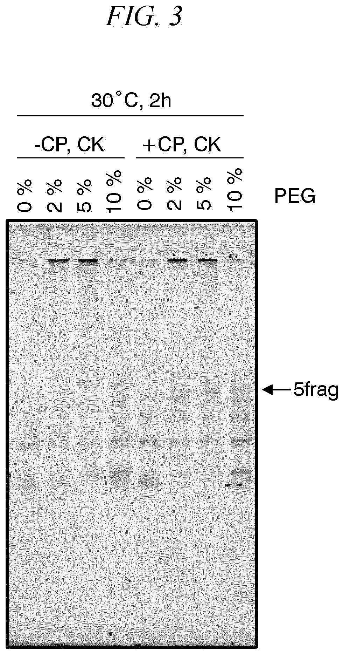

The staining results are shown in FIG. 3. In the figure, "5frag" indicats a band of a joined body in which all five fragments of Lter1 to Lter5 were joined. Among the reaction solutions without creatine phosphate and creatine kinase, in the reaction solution with 0% by weight of PEG 8000, bands of 2-fragment joined body and 3-fragment joined body were detected, but bands of joined bodies with 4 or more fragments joined were not detected. In contrast, bands of 4-fragment joined body and 5-fragment joined body were also detected in the reaction solution with high PEG 8000 concentration. On the other hand, among the reaction solutions with creatine phosphate and creatine kinase, bands of 2-fragment joined body, 3-fragment joined body, and 4-fragment joined body were detected in the reaction solutions with 0 % by weight of PEG 8000, but a band of 5-fragment joined body was not detected. In contrast, a band of 5-fragment joined body was also detected in the reaction solution with high PEG 8000 concentration. As a result of comparing the reaction solutions with 0% by mass of PEG 8000, it was found that the reaction solution with the ATP regeneration system is more likely to obtain a multi-fragment joined body. And in both the reaction solution without creatine phosphate and creatine kinase and the reaction solution with them, the production amount of the 5-fragment joined body was higher in the reaction solution with PEG than in the reaction solution without PEG From these result, it was found that PEG promotes joining. Among all the samples, it was the reaction solution with creatine phosphate, creatine kinase and 5 % by mass of PEG 8000 that had few unjoined fragments and the largest amount of 5-fragment joined body.

### [Example 3]

Two or more types of linear double-stranded DNA fragments were joined using RecA family recombinase protein and 3'→ 5' exonuclease. In the reaction, the effects of DMSO concentration in the reaction solution were investigated.

DCW1 to DCW5 (SEQ ID NO: 1 to SEQ ID NO: 5) were used as the linear double-stranded DNA fragments to be joined. The F203W mutant of E. coli RecA was used as the RecA family recombinase protein, and exonuclease III was used as the 3'→ 5' exonuclease.

Specifically, first, the reaction solutions consisting of 1 nM each of DCW1 to DCW5, 1 µM of the F203W mutant of RecA, 40 mU/µL of exonuclease III, 20 mM of Tris-HCl (pH 8.0), 4 mM of DTT, 10 mM of magnesium acetate, 100 µM of ATP, 150 mM of potassium acetate, 5 % by mass of PEG 8000, 0 %, 1 %, 3 %, or 10 % by volume of DMSO were prepared. Next, these reaction solutions were incubated at 30 °C for 30 minutes to perform the joining reaction. 2 µL of the reaction solution after the reaction was subjected to agarose gel electrophoresis, and the separated bands were stained with SYBR Green.

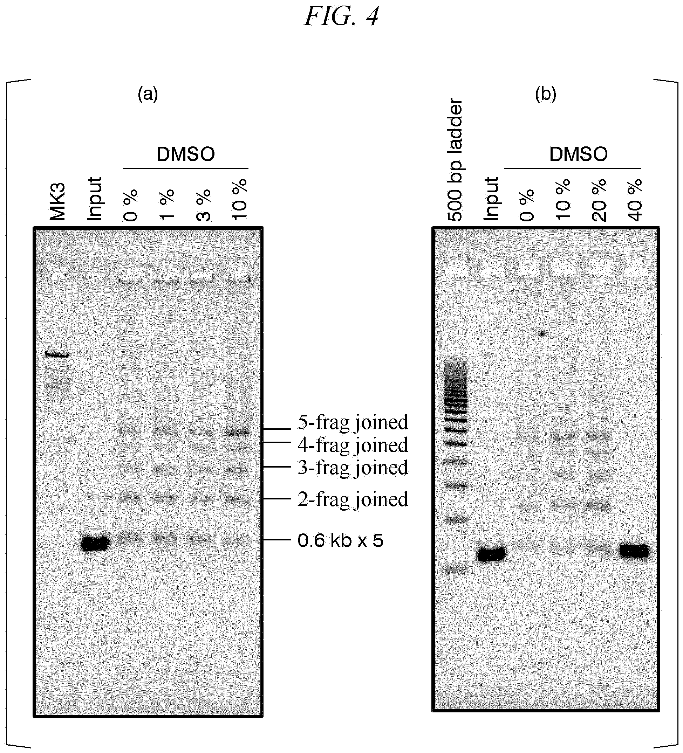

The staining results are shown in FIG. 4 (a). In the figure, "MK3" indicates the lane in which a DNA ladder marker was run, and "Input" indicates the lane in which 2 µL of the solution containing 1 nM each of DCW1 to DCW5 was run. As a result, a band of a joined body in which all five fragments were joined was detected in all the samples subjected to the joining reaction. The reaction solution with 10 % by volume of DMSO clearly had a larger content of the 5-fragment joined body than the other reaction solutions.

Next, reaction solutions were prepared in the same manner except that the DMSO concentration was 0 % by volume, 10 % by volume, 20 % by volume, or 40 % by volume and these reaction solutions were incubated at 30 °C for 30 minutes to perform the joining reaction. 2 µL of the reaction solution after the reaction was subjected to agarose gel electrophoresis, and the separated bands were stained with SYBR Green.

The staining results are shown in FIG. 4 (b). In the figure, "500 bp ladder" indicates the lane in which a DNA ladder marker used in Example 1 was run, and "Input" indicates the same as that of Fig.4 (a). As a result, the production amount of the joined body in which all five fragments were joined together was clearly increased in the reaction solution with 10 % by volume or 20 % by volume of DMSO, but the band thereof wea not detected and the joining was inhibited in the reaction solution with 40 % by volume of DMSO. The following was found from these results. The joining reaction is promoted by containing DMSO in an amount of 5% by volume or more. On the contrary, when the DMSO concentration is too high, the joining reaction is inhibited.

### [Example 4]

Two or more types of linear double-stranded DNA fragments were joined using RecA family recombinase protein and 3'→ 5' exonuclease. In the reaction, the effects of TMAC concentration in the reaction solution were investigated.

DCW1 to DCW7 (SEQ ID NO: 1 to SEQ ID NO: 7) were used as the linear double-stranded DNA fragments to be joined. The F203W mutant of E. coli RecA was used as the RecA family recombinase protein, and exonuclease III was used as the 3'→ 5' exonuclease.

Specifically, first, the reaction solutions consisting of 1 nM each of DCW1 to DCW7, 1 µM of the F203W mutant of RecA, 40 mU/µL of exonuclease III, 20 mM of Tris-HCl (pH 8.0), 4 mM of DTT, 10 mM of magnesium acetate, 100 µM of ATP, 4 mM of creatine phosphate, 20 ng/µL of creatine kinase, 150 mM of potassium glutamate, 5 % by mass of PEG8000, 10 % by volume of DMSO, and 0 mM, 15 mM, 30 mM, 60 mM, or 100 mM of TMAC were prepared. Next, these reaction solutions were incubated at 37 °C for 2 hours to perform the joining reaction. 1.5 µL of the reaction solution after the reaction was subjected to agarose gel electrophoresis, and the separated bands were stained with SYBR Green.

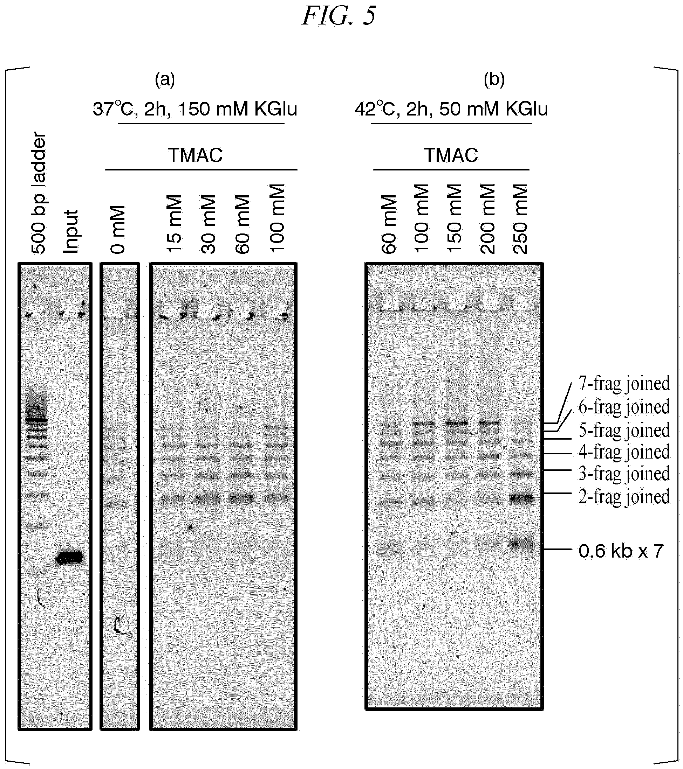

The staining results are shown in FIG. 5 (a). In the figure, "500bp ladder" indicates the lane in which a DNA ladder marker used in Example 1 was run, and "Input" indicates the lane in which 1 µL of the solution containing 1 nM each of DCW1 to DCW7 was run. As a result, a band of a joined body in which all seven fragments were joined was detected in all the samples subj ected to the joining reaction. In particular, the amount of the 7-fragment joined body in the reaction solution with 100 mM of TMAC was clearly higher than those in the other reaction solutions.

Next, reaction solutions were prepared in the same manner except that the TMAC concentration was 60 mM, 100 mM, 150 mM, 200 mM, or 250 mM and the potassium glutamate concentration was 50 mM. These reaction solutions were incubated at 42 °C for 2 hours to perform the joining reaction. 1.9 µL of the reaction solution after the reaction was subjected to agarose gel electrophoresis, and the separated bands were stained with SYBR Green.

The staining results are shown in FIG. 5 (b). As a result, the reaction solutions with 100 mM to 200 mM of TMAC clearly had a larger content of the 7-fragment joined body than the reaction solution with 60 mM of TMAC and their joining efficiency was improved. The reaction solution with 150 mM of TMAC had the largest amount of 7-fragment joined body. On the other hand, in the reaction solution with 250 mM of TMAC, the amount of the 7-fragment joined body was smaller than that in the reaction solution with 60 mM of TMAC and many unjoined fragments remained. From these result, it was found that joining is promoted by containing TMAC in an amount of 100 to 200 mM.

### [Example 5]

Two or more types of linear double-stranded DNA fragments were joined using RecA family recombinase protein and 3'→ 5' exonuclease. In the reaction, the effects of alkali metal ion sources in the reaction solution were investigated.

DCW1 to DCW7 or DCW1 to DCW5 were used as the linear double-stranded DNA fragments to be joined. The F203W mutant of E. coli RecA was used as the RecA family recombinase protein, and exonuclease III was used as the 3'→ 5' exonuclease.

Specifically, first, the reaction solutions consisting of 1 nM each of DCW1 to DCW5, 1 µM of the F203W mutant of RecA, 40 mU/µL of exonuclease III, 20 mM of Tris-HCl (pH8.0), 4 mM of DTT, 10 mM of magnesium acetate, 100 µM of ATP, 4 mM of creatine phosphate, 20 ng/µL of creatine kinase,0 mM, 50 mM, 75 mM, 100 mM, 125 mM, or 150 mM of potassium acetate, 5 % by mass of PEG 8000, and 10 % by volume of DMSO were prepared. The reaction solutions were prepared in the same manner except that 150 mM potassium glutamate was contained instead of potassium acetate. Next, these reaction solutions were incubated at 30 °C for 2 hours to perform the joining reaction. 2 µL of the reaction solution after the reaction was subjected to agarose gel electrophoresis, and the separated bands were stained with SYBR Green.

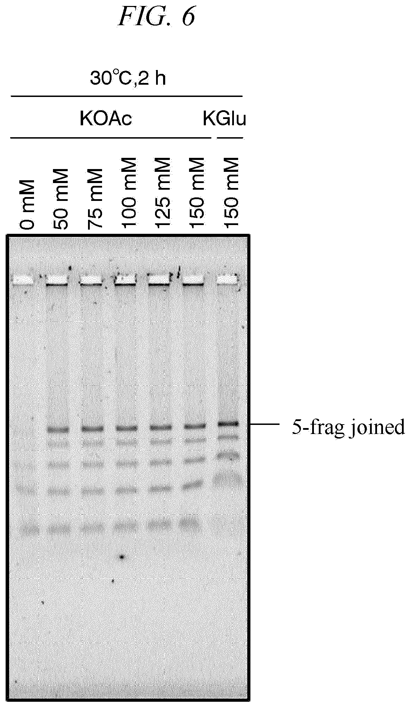

The staining results are shown in FIG 6. The band of a joined body in which all five fragments were joined was not detected in reaction solutions without alkali metal ion sources and the band was detected in all reaction solutions with potassium acetate or potassium glutamate. In particular, the band of unjoined fragments in the reaction solution with potassium glutamate was thinner than that in the reaction solution with potassium acetate. Thus, it was speculated that potassium glutamate might have a higher effect of improving the joining efficiency than potassium acetate.

Next, reaction solutions were prepared in the same manner except that DCW1 to DCW7 were used as the linear double-stranded DNA fragments to be joined, and 1 nM each of DCW 1 to DCW7 was contained, potassium glutamate was used as the alkali metal ion source, and the potassium glutamate concentration was 50 mM or 150 mM. These reaction solutions were incubated at 37 °C, 42 °C, or 45 °C for 1 hour to perform the joining reaction. 1 µL of the reaction solution after the reaction was subjected to agarose gel electrophoresis, and the separated bands were stained with SYBR Green.

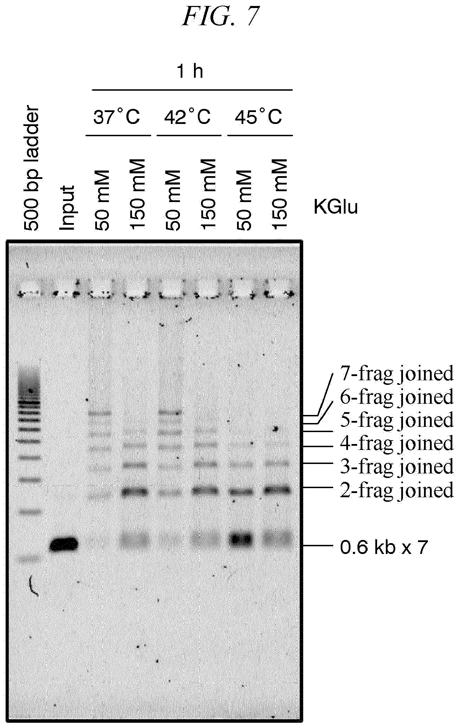

The staining results are shown in FIG 7. In the figure, "500bp ladder" indicates the lane in which a DNA ladder marker used in Example 1 was run, and "Input" indicates the lane in which 1 µL of the solution containing 1 nM each of DCW1 to DCW7 was run. As a result, at both reaction temperatures of 37 °C and 42 °C, the reaction solutions with 50 mM of potassium glutamate had higher joining efficiency than the reaction solution with 150 mM of potassium glutamate, and detected the band of a joined body in which all seven fragments were joined. From these results, it was found that the joining reaction may be inhibited when the potassium glutamate concentration is too high. In the reaction solutions incubated at 45 °C, the band of 7-fragment joined body was not detected even when the potassium glutamate concentration was 50 mM. The amount of 7-fragment joined body was the highest in the reaction solution with 50 mM of potassium glutamate and incubated at 42 °C.

### [Example 6]

Two or more types of linear double-stranded DNA fragments were joined using RecA family recombinase protein and 3'→ 5' exonuclease. In the reaction, the effects of molar ratio of each linear double-stranded DNA fragment in the reaction solution were investigated.

DCW1 to DCW7 were used as the linear double-stranded DNA fragments to be joined. The F203W mutant of E. coli RecA was used as the RecA family recombinase protein, and exonuclease III was used as the 3'→ 5' exonuclease.

Specifically, first, the reaction solutions consisting of 1 nM each of DCW1 to DCW7, 1 µM of the F203W mutant of RecA, 40 mU/µL of exonuclease III, 20 mM of Tris-HCl (pH8.0), 4 mM of DTT, 10 mM of magnesium acetate, 100 µM of ATP, 4 mM of creatine phosphate, 20 ng/µL of creatine kinase, 50 mM of potassium acetate, 5 % by mass of PEG 8000, and 10 % by volume of DMSO were prepared. The reaction solutions were prepared in the same manner except that only DCW3 concentration was 2nM. Next, these reaction solutions were incubated at 42°C for 2 hours to perform the joining reaction. 1µL of the reaction solution after the reaction was subjected to agarose gel electrophoresis, and the separated bands were stained with SYBR Green.

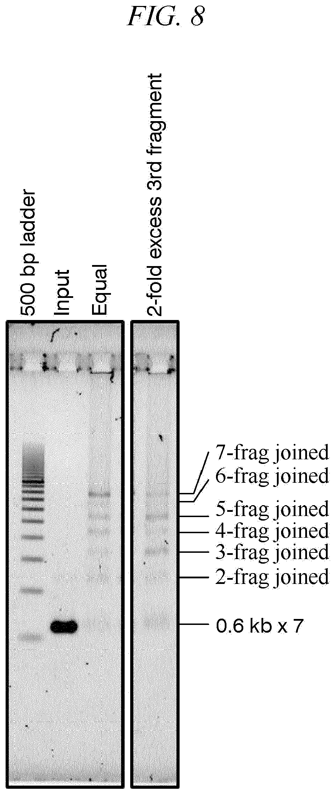

The staining results are shown in FIG 8. In the figure, "500bp ladder" indicates the lane in which a DNA ladder marker used in Example 1 was run, and "Input" indicates the lane in which 1 µL of the solution containing 1 nM each of DCW1 to DCW7 was run. "Equal" indicates the lane in which the reaction solution containing 1 nM each of DCW1 to DCW7 was run. The "2-fold excess 3^{rd} fragment" indicates the lane in which the reaction solution containing 1 nM each of DCW1 to DCW7 except DCW3 and containing 2 nM of DCW3 was run. As a result, the 7-fragment joined body was obtained in all reaction solution. However, in the reaction solution containing only twice the amount (mole) of DCW3, the amount of the 7-fragment joined body decreased and the amount of the 3-fragment joined body and the amount of the 5-ragment-joined body increased. It was speculated that excess DCW3 increased the number of joined bodies in which DCW1 to DCW3 were joined and the number of joined bodies in which DCW3 to DCW7 were jointed. From these results, it is found that the joining efficiency of multiple fragments was improved by preparing the reaction solution so that the molar ratio of each fragment to be joined was equal.

### [Example 7]

Two or more types of linear double-stranded DNA fragments were joined using RecA family recombinase protein and 3'→ 5' exonuclease. In the reaction, the effects of 3'→ 5' exonuclease concentration and reaction time in the reaction solution were investigated.

DCW1 to DCW7 were used as the linear double-stranded DNA fragments to be joined. The wild-type of E. coli RecA was used as the RecA family recombinase protein, and exonuclease III was used as the 3'→ 5' exonuclease.

Specifically, first, the reaction solutions consisting of 1 nM each of DCW1 to DCW7, 1 µM of the wild-type of E. coli RecA, 20 mU/µL, 40 mU/µL, 80 mU/µL, 120 mU/µL, or 160 mU/µL of exonuclease III, 20 mM of Tris-HCl (pH8.0), 4 mM of DTT, 1mM of magnesium acetate, 100 µM of ATP, 4 mM of creatine phosphate, 20 ng/µL of creatine kinase, 50 mM of potassium glutamate, 150 mM of TMAC, 5 % by mass of PEG 8000, and 10 % by volume of DMSO were prepared. Next, these reaction solutions were incubated at 42 °C for 15 minutes, 30 minutes, or 60 minutes to perform the joining reaction. 1 µL of the reaction solution after the reaction was subjected to agarose gel electrophoresis, and the separated bands were stained with SYBR Green.

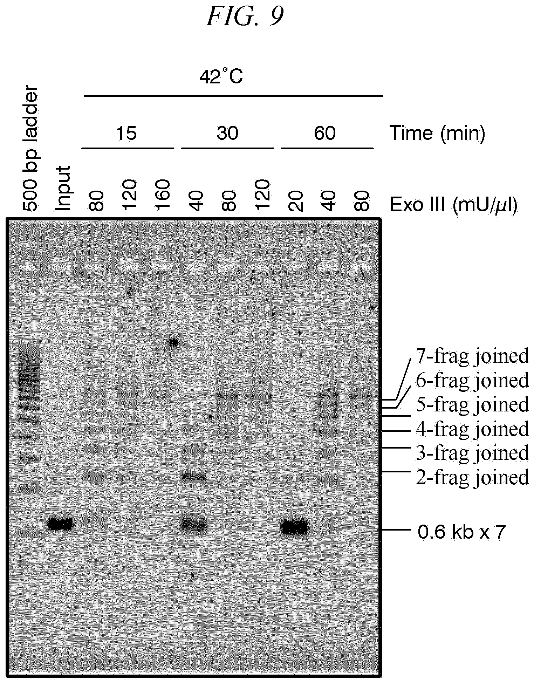

The staining results are shown in FIG 9. In the figure, "500bp ladder" indicates the lane in which a DNA ladder marker used in Example 1 was run, and "Input" indicates the lane in which 1 µL of the solution containing 1 nM each of DCW1 to DCW7 was run. As a result, in the reaction solutions with 20 mU/µL, almost no joined body was formed even when the incubation time was 60 minutes. In contrast, in the reaction solutions with 40 mU/µL of exonuclease III, almost no joined body was formed at the incubation time of 30 minutes, but the 7-fragment joined body was formed at the incubation time of 60 minutes. In the reaction solutions with 80 to 160 mU/µL of exonuclease III, the 7-fragment joined body was formed even at the incubation time of 15 minutes. The reaction solution with 80 mU/µL of exonuclease III and incubated for 30 minutes had the largest amount of 7-fragment joined body, and the best joining efficiency of multiple fragments.

### [Example 8]

Two or more types of linear double-stranded DNA fragments were joined using RecA family recombinase protein and 3'→ 5' exonuclease. In the reaction, the effects of linear double-stranded DNA fragment concentration in the reaction solution were investigated.

DCW1 to DCW49 (SEQ ID NO: 1 to SEQ ID NO: 49) were used as the linear double-stranded DNA fragments to be joined. Similar to DCW1 to DCW7, the regions of DCW8 to DCW49 from each end to 60 bases were homologous regions. The wild-type of E. coli RecA was used as the RecA family recombinase protein, and exonuclease III was used as the 3'→ 5' exonuclease.

Specifically, first, the reaction solutions consisting of 1 nM or 0.5 nM each of linear double-stranded DNA fragments, 1 µM of the wild-type of E. coli RecA, 80 mU/µL of exonuclease III, 20 mM of Tris-HCl (pH8.0), 4 mM of DTT, 1mM of magnesium acetate, 100 µM of ATP, 4 mM of creatine phosphate, 20 ng/µL of creatine kinase, 50 mM of potassium glutamate, 150 mM of TMAC, 5 % by mass of PEG 8000, and 10 % by volume of DMSO were prepared. In the reaction solution containing 1 nM each of DCW1 to DCW20, the total amount of linear double-stranded DNA fragments was 20nM (7.8ng/µL). In the reaction solution containing 1 nM each of DCW1 to DCW25, the total amount of linear double-stranded DNA fragments was 25 nM (9.8ng/µL). In the reaction solution containing 1 nM each of DCW1 to DCW30, the total amount of linear double-stranded DNA fragments was 30 nM (11.7 ng/µL). In the reaction solution containing 1 nM each of DCW1 to DCW40, the total amount of linear double-stranded DNA fragments was 40 nM (15.6 ng/µL). In the reaction solution containing 1 nM each of DCW1 to DCW49, the total amount of linear double-stranded DNA fragments was 49 nM (19.1 ng/µL). In the reaction solution containing 0.5 nM each of DCW1 to DCW49, the total amount of linear double-stranded DNA fragments was 24.5 nM (9.6 ng/µL). Next, these reaction solutions were incubated at 42 °C for 30 minutes to perform the joining reaction. The following volume of the reaction solution after the reaction was subjected to agarose gel electrophoresis, and the separated bands were stained with SYBR Green. The volume of the reaction solution was, 1.25 µL for the reaction solution using 1 nM each of DCW1 to DCW20, 1 µL for the reaction solution using 1nM each of DCW1 to DCW25, and 0.83 µL for the reaction solution using 1 nM each of DCW1 to DCW30, 0.63 µL of the reaction solution using 1 nM each of DCW1 to DCW40, 0.51 µL of the reaction solution using 1 nM each of DCW1 to DCW49, and 1.02 µL of the reaction solution using 0.5 nM each of DCW1 to DCW49.

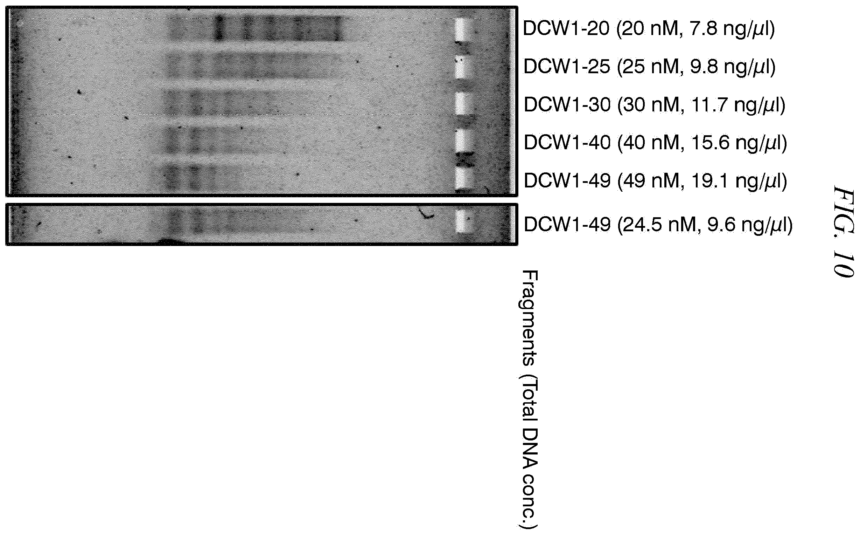

The staining results are shown in FIG 10. In the reaction solutions containing 1nM each of linear double-stranded DNA fragments, it became more difficult to form a multi-fragment joined body, as the number of contained fragments increased, that is, the total amount of linear double-stranded DNA fragments in the reaction solution increased. Compared to the reaction solutions containing DCW1 to DCW49, the joined bodies that were joined a larger number of fragments were formed in the reaction solution containing 0.5nM each of linear double-stranded DNA fragments than in the reaction solution containing 1nM each of linear double-stranded DNA fragments. These results suggested that the joining efficiency may be inhibited by too much total amount of linear double-stranded DNA fragments in the reaction solution.

### [Example 9]

Two or more types of linear double-stranded DNA fragments were joined using RecA family recombinase protein and 3'→ 5' exonuclease. In the reaction, the effects of type of RecA family recombinase protein in the reaction solution were investigated.

DCW1 to DCW25 (SEQ ID NO: 1 to SEQ ID NO: 25) were used as the linear double-stranded DNA fragments to be joined. The wild-type or F203W mutant of E. coli RecA was used as the RecA family recombinase protein, and exonuclease III was used as the 3'→ 5' exonuclease.

Specifically, first, the reaction solutions consisting of 1 nM each of DCW1 to DCW25, 0.5 µM, 0.75 µM, 1 µM, 1.25 µM, or 1.5 µM of the wild-type or F203W mutant of E. coli RecA, 80 mU/µL of exonuclease III, 20 mM of Tris-HCl (pH8.0), 4 mM of DTT, 1 mM of magnesium acetate, 100 µM of ATP, 4 mM of creatine phosphate, 20 ng/µL of creatine kinase, 50 mM of potassium glutamate, 150 mM of TMAC, 5 % by mass of PEG 8000, and 10 % by volume of DMSO were prepared. Next, these reaction solutions were incubated at 42 °C for 30 minutes to perform the joining reaction, and then incubated at 65 °C for 20 minutes. After the incubation at 65 °C and rapidly cooled on ice, 1.5 µL of the reaction solution after the reaction was subjected to agarose gel electrophoresis, and the separated bands were stained with SYBR Green.

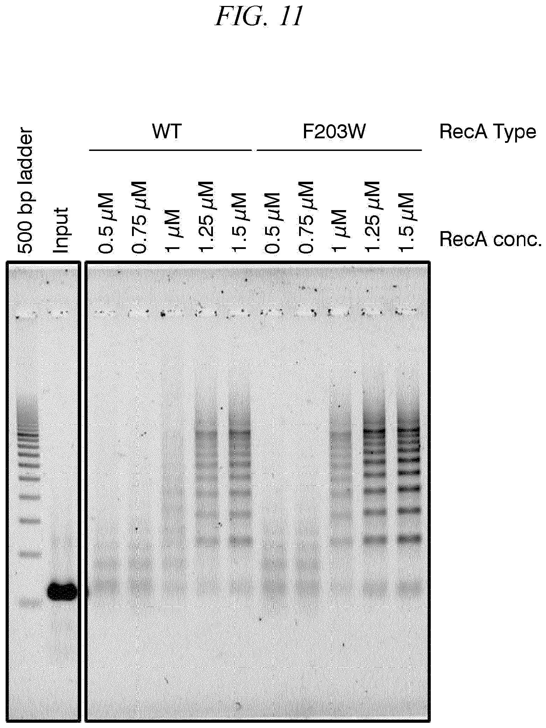

The staining results are shown in FIG 11. In the figure, "500bp ladder" indicates the lane in which a DNA ladder marker used in Example 1 was run, and "Input" indicates the lane in which 1.5 µL of the solution containing 1 nM each of DCW1 to DCW25 was run. As a result, whether the RecA family recombinase protein was the wild-type or F203W mutant, the production amount of the multi-fragment joined bodies was increased depending on the RecA content. The reaction solution with the F203W mutant had a higher production amount of multi-fragment joined bodies and higher joining efficiency than the reaction solution with the wild-type RecA.

### [Example 10]

Two or more types of linear double-stranded DNA fragments were joined using RecA family recombinase protein and 3'→ 5' exonuclease to form a circular joined body, and the circular joined body was RCR amplified.

First, a set of DCW1 to DCW20 (SEQ ID NO: 1 to SEQ ID NO: 20) and Cm-oriC (DCW20) (SEQ ID NO: 50) which contained oriC and a pair of the ter sequences inserted outwardly with respect to oriC respectively was used as the linear double-stranded DNA fragments to be joined. The ter sequence is a sequence to which Tus protein binds. Tus protein has a function of stopping replication in a direction-specific manner. "Inserting outwardly with respect to oriC" for a ter sequence meant that the ter sequence is inserted such that, by the action of a combination of proteins that bind to the ter sequence to inhibit replication, replication in a direction outward from oriC is allowed and replication in a direction entering toward oriC is not allowed and stop. Cm-oriC (DCW20) was a linear double-stranded DNA fragment of 1298 bp, and the region of 60 bases from the first to the 60^{th} was the homologous region for joining with DCW20, which consisted of the same base sequence as 60 bases from 532^{rd} to 591^{st} of DCW20. The region of 60 bases from 1239^{th} to 1298^{th} of Cm-oriC (DCW20) was the region for joining with DCW1, which consisted of the same base sequence as 60 bases from the first to the 60^{th} of DCW1. That is, when all 21 fragments of DCW1 to DCW20 and Cm-oriC (DCW20) were joined, circular DNA was obtained.

In addition, a set of DCW1 to DCW25 (SEQ ID NO: 1 to SEQ ID NO: 25) and Cm-oriC (DCW25) (SEQ ID NO: 51) including oriC was used as the linear double-stranded DNA fragments to be joined. Cm-oriC (DCW25) was a linear double-stranded DNA fragment of 1298bp, and the region of 60 bases from the first to the 60^{th} was the homologous region for joining with DCW25, which consisted of the same base sequence as 60 bases from 532^{rd} to 591^{st} of DCW25. The region of 60 bases from 1239^{th} to 1298^{th} of Cm-oriC (DCW25) was the region for joining with DCW1, which consisted of the same base sequence as 60 bases from the first to the 60^{th} of DCW1. That is, when all 26 fragments of DCW1 to DCW25 and Cm-oriC (DCW25) were joined, circular DNA was obtained.

The F203W mutant of E. coli RecA was used as the RecA family recombinase protein, and exonuclease III was used as the 3'→ 5' exonuclease. Furthermore, as the RCR amplification reaction solution, a mixture solution containing 60nM of Tus in the reaction mixture having the composition shown in Table 1 was used. Tus was prepared and purified from an E. coli expression strain of Tus in a process including affinity column chromatography and gel filtration column chromatography.

**[Table 1]**

| Reaction mixture | | | |
|---|---|---|---|
| | Reaction buffer | | |
| | | Tris-HCl (pH8.0) | 20 mM |
| | | Dithiothreitol | 8 mM |
| | | Potassium glutamate | 150 mM |
| | | Mg(OAc)₂ | 10 mM |
| | | Creatine phosphate | 4 mM |
| | | ATP | 1 mM |
| | | GTP, CTP, UTP | each 1 mM |
| | | dNTPs | each 0.1 mM |
| | | tRNA | 50 ng/µL |
| | | NAD | 0.25 mM |
| | | Ammonium sulfate | 10 mM |
| | | Bovine serum albumin (BSA) | 0.5 mg/mL |
| | | Creatine kinase | 20 ng/µL |

| | Enzymes | | |
|---|---|---|---|
| | | SSB | 400 nM |
| | | 1HF | 20 nM |
| | | DnaG | 400 nM |
| | | DnaN | 40 nM |
| | | PolIII* | 5 nM |
| | | DnaB, DnaC | 20 nM |
| | | DnaA | 100 nM |
| | | RNaseH | 10 nM |
| | | Ligase | 50 nM |
| | | PolI | 50 nM |
| | | GyrA, GyrB | 50 nM |
| | | Topo IV | 5 nM |
| | | Topo III | 50 nM |
| | | RecQ | 50 nM |

In Table 1, SSB represented an E. coli-derived SSB, IHF represented a complex of E. coli-derived IhfA and IhfB, DnaG represented an E. coli-derived DnaG, DnaN represented an E. coli-derived DnaN, Pol III * represented a DNA polymerase III ^{∗} complex that was a complex composed of E. coli-derived DnaX, HolA, HolB, HolC, HolD, DnaE, DnaQ, and HolE, DnaB represented E. coli-derived DnaB, DnaC represented E. coli-derived DnaC, DnaA represented E. coli-derived DnaA, RNaseH represented E. coli-derived RNaseH, Ligase represented E. coli-derived DNA ligase, Pol I represented E. coli-derived DNA polymerase I, GyrA represented E. coli-derived GyrA, GyrB represented E. coli-derived GyrB, Topo IV represented a complex of E. coli-derived ParC and ParE, Topo III represented E. coli-derived topoisomerase III, RecQ Represented E. coli-derived RecQ.

SSB was purified and prepared from an E. coli expression strain of SSB by a process including ammonium sulfate precipitation and ion exchange column chromatography.

IHF was prepared from IhfA and IhfB co-expressing E. coli strains by purification including ammonium sulfate precipitation and affinity column chromatography.

DnaG was prepared by purifying from an E. coli expression strain of DnaG in steps including ammonium sulfate precipitation, anion exchange column chromatography, and gel filtration column chromatography.

DnaN was purified and prepared from an E. coli expression strain of DnaN in a process including ammonium sulfate precipitation and anion exchange column chromatography.

Pol III * was purified and prepared from E. coli co-expressing strains of DnaX, HolA, HolB, HolC, HolD, DnaE, DnaQ, and HolE in steps including ammonium sulfate precipitation, affinity column chromatography, and gel filtration column chromatography

DnaB and DnaC were purified and prepared from E. coli co-expressing strains of DnaB and DnaC in steps including ammonium sulfate precipitation, affinity column chromatography, and gel filtration column chromatography.

DnaA was purified and prepared from an Escherichia coli expression strain of DnaA in steps including ammonium sulfate precipitation, dialysis precipitation, and gel filtration column chromatography.

GyrA and GyrB were purified and prepared from a mixture of an E. coli expression strain of GyrA and an E. coli expression strain of GyrB by a process including ammonium sulfate precipitation, affinity column chromatography, and gel filtration column chromatography.

Topo IV was prepared from a mixture of an Escherichia coli expression strain of ParC and an Escherichia coli expression strain of ParE by a process including ammonium sulfate precipitation, affinity column chromatography, and gel filtration column chromatography.

Topo III was prepared from a E. coli-expressing strain of Topo III by purification in a process including ammonium sulfate precipitation and affinity column chromatography.

RecQ was prepared by purifying from an Escherichia coli expression strain of RecQ in steps including ammonium sulfate precipitation, affinity column chromatography, and gel filtration column chromatography.

RNaseH, Ligase, and Pol I used commercially available enzymes derived from E. coli (manufactured by Takara Bio Inc.).

Specifically, first, the reaction solutions consisting of 2.5 nM or 5 nM of the set of linear double-stranded DNA fragments, 1 µM of the F203W mutant of RecA, 80 mU/µL of exonuclease III, 20 mM of Tris-HCl (pH8.0), 4 mM of DTT, 1 mM of magnesium acetate, 100µM of ATP, 4 mM of creatine phosphate, 20 ng/µL of creatine kinase, 50 mM of potassium glutamate, 5 % by mass of PEG 8000, and 10 % by volume of DMSO were prepared. As the set of linear double-stranded DNA fragments, the set containing all equimolar amounts of DCW 1 to DCW20 and Cm-oriC (DCW20) or the set containing all equimolar amounts of DCW1 to DCW25 and Cm-oriC (DCW25) was used. Next, these reaction solutions were incubated at 42 °C for 30 minutes to perform the joining reaction, then incubated at 65 °C for 20 minutes for heat treatment, and then rapidly cooled on ice. After the heat treatment and rapid cooling, 1µL of the reaction solutions containing 2.5 nM of the set of the linear double-stranded DNA fragment and 0.5 µL of the reaction solutions containing 5 nM of the set of the linear double-stranded DNA fragments were subjected to agarose gel electrophoresis, and the separated bands were stained with SYBR Green.

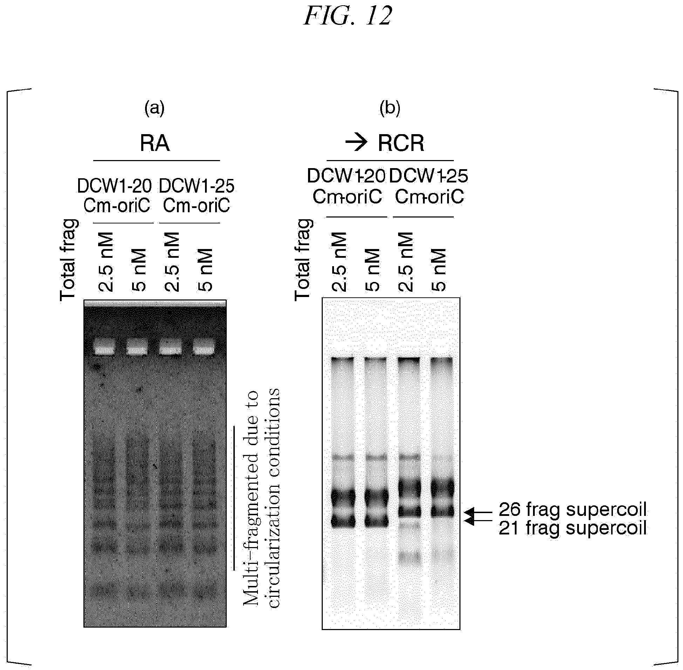

The staining results are shown in FIG 12 (a). In the figure, "1-20" indicates the lane in which the solution containing the set of linear double-stranded DNA fragments with all equimolar amounts of DCW1 to DCW20 and Cm-oriC (DCW20) was run. The "1-25" indicates the lane in which the solution containing the set of linear double-stranded DNA fragments with all equimolar amounts of DCW1 to DCW25 and Cm-oriC (DCW25) was run. As a result, it was confirmed that all sizes of the joining bodies were contained in all reaction solutions.

Next, reaction mixtures were prepared by adding 0.5 µL of the reaction solution after the heat treatment and rapid cooling to 4.5 µL of the RCR amplification reaction solution. The reaction mixtures were incubated at 30 °C for 13 hours to perform the RCR amplification. 1 µL of the reaction mixture after the reaction was subjected to agarose gel electrophoresis, and the separated band was stained with SYBR Green.

The staining results are shown in FIG 12 (b). In the figure, "1-20"and "1-25" indicates the same as those in FIG 12 (a). As a result, in the lane of the RCR amplification product of the reaction solution in which the set of DCW1 to DCW20 and Cm-oriC (DCW20) were joined, a band of a supercoiled form of a circular 21-fragment joined body ("21 frag supercoil" in the figure) was detected. In the lane of the RCR amplification product of the reaction solution in which the set of DCW1 to DCW25 and Cm-oriC (DCW25) were joined, a band of a supercoiled form of a circular 26-fragment joined body ("26 frag supercoil" in the figure) was detected. While many bands were detected in the reaction solutions after the joining reaction (FIG. 12 (a)), only a few bands were detected in the reaction mixture after RCR amplification (FIG. 12 (b)). It was confirmed that only circular joined bodies were amplified by RCR amplification.

### [Example 11]

Two or more types of linear double-stranded DNA fragments were joined using RecA family recombinase protein and 3'→ 5' exonuclease to form a circular joined body, and the resulting circular joined body was RCR amplified. In this reaction, the effects of the heat treatment and rapid cooling before RCR amplification were examined.

As the linear double-stranded DNA fragments to be joined , the set of linear double-stranded DNA fragments with all equimolar amounts of DCW1 to DCW25 and Cm-oriC (DCW25) (referred to as "a set of 20nM of linear double-stranded DNA fragment") used in Example 10 was used. The F203W mutant of E. coli RecA was used as the RecA family recombinase protein, and exonuclease III was used as the 3'→ 5' exonuclease. Furthermore, as the RCR amplification reaction solution, a mixture solution containing 60nM of Tus in the reaction mixture having the composition shown in Table 1 was used.

Specifically, first, the reaction solutions consisting of 20nM of the set of linear double-stranded DNA fragment, 1.5µM of the F203W mutant of RecA, 80mU/µL of exonuclease III, 20mM of Tris-HCl (pH8.0), 4mM of DTT, 1mM of magnesium acetate, 100µM of ATP, 4mM of creatine phosphate, 20ng/µL of creatine kinase, 50mM of potassium glutamate, 150mM of TMAC, 5% by mass of PEG 8000, and 10% by volume of DMSO were prepared. Next, these reaction solutions were incubated at 42°C for 30 minutes to perform the joining reaction, then incubated at 50°C or 65°C for 2 minutes for heat treatment, and then rapidly cooled on ice. 1.5µL of the reaction solutions after rapid cooled were subjected to agarose gel electrophoresis, and the separated bands were stained with SYBR Green.

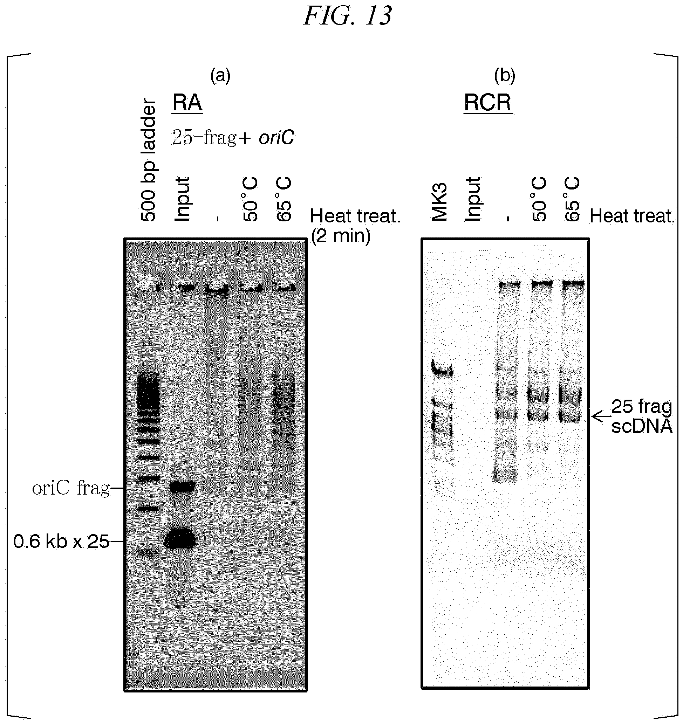

The staining results are shown in FIG 13 (a). In the figure, "500bp ladder" indicates the lane in which a DNA ladder marker used in Example 1 was run, and the "Input" indicates the lane in which 1.5 µL of the solution containing 20 nM of the set of linear double-stranded DNA fragment was run. The "-" indicates the lane in which the reaction solution that was not heat-treated after the joining reaction was run. The "50 °C" indicates the lane in which the reaction solution that was heat-treated at 50 °C for 2 minutes was run. The "65 °C" indicates the lane in which the reaction solution that was heat-treated at 65 °C for 2 minutes was run. As shown in FIG 13 (a), in the reaction solutions without heat-treatment, DNA were not migrated to be a smear band, whereas in the reaction solutions with heat-treatment, most of the smear band were eliminated.

Next, reaction mixtures were prepared by adding 0.5 µL of the reaction solution after the heat treatment and rapid cooling to 4.5 µL of the RCR amplification reaction solution. The reaction mixtures were incubated at 30 °C for 13 hours to perform the RCR amplification. As a control, 0.5 µL of the reaction solution after heat treatment at 65°C and rapid cooling was added to 4.5 µL of TE solution composed of 10 mM Tris-HCl (pH 8.0) and 1 mM EDTA to prepare a pre-amplification solution. 1 µL of the the pre-amplification solution and reaction mixture after the reaction was subjected to agarose gel electrophoresis, and the separated bands were stained with SYBR Green.

The staining results are shown in FIG 13 (b). In the figure, the "MK3" indicates the lane in which a DNA ladder marker was run. As a result, in the reaction mixture obtained by RCR amplification of the reaction solution in which circular joined bodies were formed by the joining reaction, a band of a supercoiled form of a circular 26-fragment joined body ("25 frag scDNA" in the figure) was detected ("-", "50 °C", "65 °C" in FIG 13 (b)). No band was detected in the pre-amplification solution ("Input" in FIG 13 (b)). Two broad bands where the migration distance was longer than the band of the supercoiled 26-fragment joined body were detected in the reaction mixture without heat-treatment ("-"), whereas those bands were thin in the reaction mixture with heat-treatment at 50 °C. ("50 °C"), and were not detected in the reaction mixture with heat-treatment at 65 °C. ("65 °C"). From these results, it was found that DNA of bands where the migration distance was longer than the band of the supercoiled 26-fragment joined body were the amplification products of circular joined bodies obtained by non-specific joining, and such non-specific amplification products can be suppressed by the heat treatment and rapid cooling prior to RCR amplification.

### [Example 12]

26 or 36 types of linear double-stranded DNA fragments were joined using RecA family recombinase protein and 3'→ 5' exonuclease to form a circular joined body, and the circular joined body was RCR amplified.

As the linear double-stranded DNA fragments to be joined, the set of DCW1 to DCW25 (SEQ ID NO: 1 to SEQ ID NO: 25) and Km-oriC (DCW25) (SEQ ID NO: 52) which contained oriC was used. Km-oriC (DCW25) was a linear double-stranded DNA fragment of 1509 bp, and the region of 60 bases from the first to the 60^{th} was the homologous region for joining with DCW25, which consisted of the same base sequence as 60 bases from 532^{rd} to 591^{st} of DCW25. The region of 60 bases from 1450^{th} to 1509^{th} of Km-oriC (DCW25) was the region for joining with DCW1, which consisted of the same base sequence as 60 bases from the first to the 60^{th} of DCW1. That is, circular DNA was obtained by joining all 26 types of fragments of DCW1 to DCW25 and Km-oriC (DCW25).

As the linear double-stranded DNA fragments to be joined, the set of DCW1 to DCW35 (SEQ ID NO: 1 to SEQ ID NO: 35) and Km-oriC (DCW35) (SEQ ID NO: 53) which contained oriC and a pair of the ter sequences inserted outwardly with respect to oriC respectively was used. Km-oriC (DCW35) was a linear double-stranded DNA fragment of 1509 bp, and the region of 60 bases from the first to the 60^{th} was the homologous region for joining with DCW35, which consisted of the same base sequence as 60 bases from 532^{rd} to 591^{st} of DCW35. The region of 60 bases from 1450^{th} to 1509^{th} of Km-oriC (DCW35) was the region for joining with DCW1, which consisted of the same base sequence as 60 bases from the first to the 60^{th} of DCW1. That is, a circular DNA was obtained by joining all 36 types of fragments of DCW1 to DCW35 and Km-oriC (DCW35).

The F203W mutant of E. coli RecA was used as the RecA family recombinase protein, and exonuclease III was used as the 3'→ 5' exonuclease. Furthermore, as the RCR amplification reaction solution, a mixture solution containing 60nM of Tus in the reaction mixture having the composition shown in Table 1 was used.

Specifically, first, the reaction solutions consisting of 20nM of the set of linear double-stranded DNA fragments, 1.5µM of the F203W mutant of RecA, 80mU/µL of exonuclease III, 20mM of Tris-HCl (pH8.0), 4mM of DTT, 1mM of magnesium acetate, 100µM of ATP, 4mM of creatine phosphate, 20ng/µL of creatine kinase, 50mM of potassium glutamate, 150mM of TMAC, 5% by mass of PEG 8000, and 10% by volume of DMSO were prepared. As the set of linear double-stranded DNA fragments, the set containing all equimolar amounts of DCW1 to DCW25 and Cm-oriC (DCW25) or the set containing all equimolar amounts of DCW1 to DCW35 and Cm-oriC (DCW35) was used. Next, these reaction solutions were incubated at 42°C for 30 minutes to perform the joining reaction, then incubated at 65°C for 5 minutes for heat treatment, and then rapidly cooled on ice. After the heat treatment and rapid cooling, 1.5µL of the reaction solutions were subjected to agarose gel electrophoresis, and the separated bands were stained with SYBR Green.

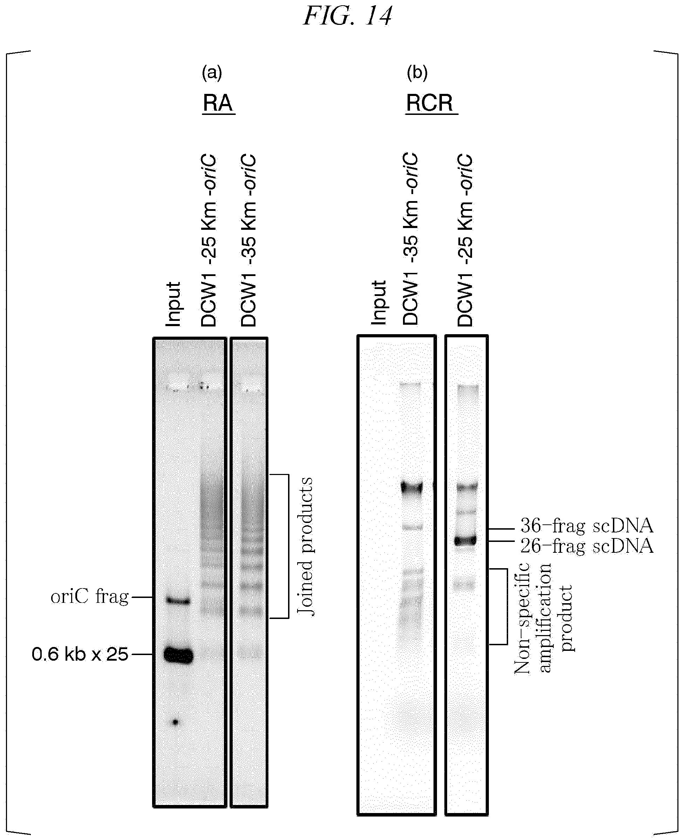

The staining results are shown in FIG 14 (a). In the figure, "Input" indicates the lane in which 1.5 µL of the solution containing the set of 20 nM of linear double-stranded DNA fragments was run. The "DCW1-25 Km-oriC" indicates the lane in which the reaction solution containing the set with all equimolar amounts of DCW1 to DCW25 and Km-oriC (DCW25) was run. The "DCW1-35 Km-oriC" indicates the lane in which the reaction solution containing the set with all equimolar amounts of DCW1 to DCW35 and Km-oriC (DCW35) was run. As shown in FIG 14 (a), when any set of linear double-stranded DNA fragments was used, multi-fragment joined bodies were obtained by the joining reaction.

Next, 0.5µL of the reaction solution after the heat treatment and rapid cooling was added to 4.5µL of the RCR amplification reaction solution to prepare a reaction mixture. RCR amplification reaction was performed by incubating the reaction mixture at 30°C for 16 hours. Subsequently, 0.5µL of each RCR amplification reaction product was diluted to 4.5µL by the reaction buffer, which was obtained by removing only the enzyme group from the reaction mixture shown in Table 1, and then re-incubated at 30°C for 30 minutes. The re-incubation treatment after dilution has the effect of promoting the replication extension and separation reaction of the amplification intermediate in the product and increasing the production amount of the supercoiled DNA that is the final product. 0.5µL of the reaction solution in which a joining reaction is performed using DCW1 to DCW25, followed by the heat treatment and rapid cooling was added to 4.5µL of TE solution consisting of 10mM Tris-HCl (pH 8.0) and 1mM EDTA. This solution thus prepared was used as a control. The solution thus prepared was used as a control for the pre-amplification solution. 2.5µL of the pre-amplification solution and the reaction mixture after the reincubation were subjected to agarose gel electrophoresis, and the separated bands were stained with SYBR Green.

The staining results are shown in FIG 14 (b). As a result, in the reaction mixture obtained by RCR amplification after joining of the set of linear double-stranded DNA fragments containing 26 fragments, a band of a supercoiled form of a circular 26-fragment joined body ("26-frag scDNA" in the figure) was detected ("DCW1-25 Km-oriC" in the FIG 13 (b)). In the reaction mixture obtained by RCR amplification after joining of the set of linear double-stranded DNA fragments containing 36 fragments, a band of a supercoiled form of a circular 36-fragment joined body ("36-frag scDNA" in the figure) was detected ("DCW1-35 Km-oriC" in the FIG 13 (b)). No band was detected in the pre-amplification solution ("Input" in FIG 14 (b)). From these results, it was confirmed that a circular multi-fragment joined body of 36 fragments can be obtained by the present invention, and that this circular joined body can be amplified by RCR amplification. However, the 36-fragment joined body had more non-specific amplification products by RCR amplification than the 26-fragment joined body.

### [Example 13]

A kit "NEBuilder HiFi DNA assembly" (manufactured by NEB) used in a method for joining a plurality of double-stranded DNA fragments using the Gibson Assembly method (Patent Literature 3) was commercially available. In the kit, two or more types of linear double-stranded DNA fragments having a homologous region of 15 to 20 bases at the end were joined by the NEB method, that is, adding the DNA fragments into the mixed solution (Master mix), and then incubating the mixed solution at 50°C for 15 to 60 minutes. The mixed solution was included with the kit and contained 5'→ 3' exonuclease, DNA polymerase, and DNA ligase.

The joining efficiency of the DNA production method according to the present invention in which a joining reaction was performed using RecA family recombinase protein and 3'→ 5' exonuclease was compared with that of the NEB method.

As the linear double-stranded DNA fragments to be joined, the set containing all equimolar amounts of DCW1 to DCW25 (SEQ ID NO: 1 to SEQ ID NO: 25) and Km-oriC (DCW25) which were used in the Example 12 was used. The F203W mutant of E. coli RecA was used as the RecA family recombinase protein, and exonuclease III was used as the 3'→ 5' exonuclease. Furthermore, as the RCR amplification reaction solution, a mixture solution containing 60nM of Tus in the reaction mixture having the composition shown in Table 1 was used.

Specifically, first, the reaction solutions consisting of 20nM or 60nM of the set of linear double-stranded DNA fragments, 1.5µM of the F203W mutant of RecA, 80mU/µL of exonuclease III, 20mM of Tris-HCl (pH8.0), 4mM of DTT, 1mM of magnesium acetate, 100µM of ATP, 4mM of creatine phosphate, 20ng/µL of creatine kinase, 50mM of potassium glutamate, 150mM of TMAC, 5% by mass of PEG 8000, and 10% by volume of DMSO were prepared for a method according to the present invention (RA method). Next, these reaction solutions were incubated at 42°C for 30 minutes to perform the joining reaction, then incubated at 65°C for 5 minutes for heat treatment, and then rapidly cooled on ice. 1.5µL of the reaction solutions after the rapid cooling were subjected to agarose gel electrophoresis, and the separated bands were stained with SYBR Green.

As the NEB method, a reaction solution was prepared by mixing 20nM or 60nM of the set of linear double-stranded DNA fragment with a solution diluted 2-fold with the "2 × Master mix" attached to the said kit. The reaction solution was incubated at 50 ° C. for 60 minutes to perform the j oinig reaction. 1.5µL of the reaction solution after the joining reaction was subjected to agarose gel electrophoresis, and the separated bands were stained with SYBR Green.

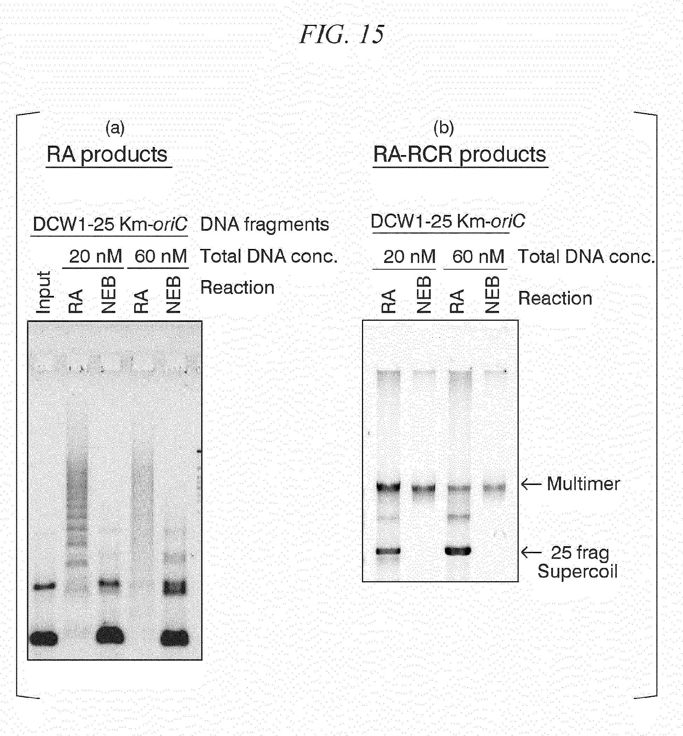

The staining results are shown in FIG 15 (a). In the figure, the "Input" indicates the lane in which 1.5µL of the solution containing the 20nM of the set of linear double-stranded DNA fragments was run. "RA" indicates the lane in which the reaction solution prepared by the method according to the present invention (the RA method) was run. "NEB" indicates the lane in which the reaction solution prepared by the NEB method was run. As shown in FIG 15 (a), when the joining reaction was performed by the RA method, a considerable number of fragments were joined regardless of whether the linear double-stranded DNA fragment set content of the reaction solution was 20 nM or 60 nM. On the other hand, in the reaction solution in which the joining reaction was performed by the NEB method, only joined bodies of 2 to 3 fragments were obtained.

Next, reaction mixtures were prepared by adding 0.5µL of the reaction solution after the heat treatment and rapid cooling to 4.5µL of the RCR amplification reaction solution. The reaction mixtures were incubated at 30°C for 16 hours to perform the RCR amplification. Subsequently, 0.5µL of each RCR amplification reaction product was diluted to 4.5µL by the reaction buffer, which was obtained by removing only the enzyme group from the reaction mixture shown in Table 1, and then re-incubated at 30°C for 30 minutes. 2.5µL of the reaction mixture after the re-incubation was subjected to agarose gel electrophoresis, and the separated bands were stained with SYBR Green.

The staining results are shown in FIG 15 (b). As a result, in the reaction mixture obtained by RCR amplification after joining by the method according to the present invention (the RA method) ("RA" in the figure), a band of a supercoiled form of a circular joined body in which all 26 fragments were joined ("25 frag Supercoil" in the figure) was detected. On the otherhand, in the reaction mixture obtained by RCR amplification after joining by the NEB method ("NEB" in the figure), a band of a circular 26-fragment joined body was not detected and 26 fragments could not be joined by the NEB method. In both reaction mixtures, products that became concatemers due to the progress of non-specific rolling circle replication, and circular DNA multimer products (catenanes) that remained unseparated after replication were also detected at the separation limit of agarose gel electrophoresis ("Multimer" in the figure).

### [Example 14]

Long genome fragments were joined together to form a circular joined body, and the circular joined body was RCR amplified.

An Xba I digest (15 fragments, DGF-298 / XbaI) of genomic DNA of an E. coli strain (DGF-298WΔ100:: revΔ234 :: SC) was used as a long-chain genomic fragment. Of these digests, a 325kbp genomic fragment (325k-genomic fragment) and a 220kbp genomic fragment (220k-genomic fragment) were each joined with a joining fragment containing oriC (Cm-oriC fragment) to form a circular shape. As a joining fragment for cyclizing the 325k-genomic fragment, 1298 bp of a linear double-stranded DNA fragment including oriC (Cm-oriC / 325k fragment, SEQ ID NO: 59) was ued. The upstream end region of this joining fragment was homologous to the downstream end of the 325k-genomic fragment (that is, the 60 bases at the upstream end of this fragment consisted of the same base sequence as the 60 bases at the downstream end), and the downstream end region of the joining fragment was homologous to the upstream end of the 325k-genomic fragment (that is, the 60 bases at the downstream end of this fragment consisted of the same base sequence as the upstream 60 bases). As a joining fragment for cyclizing the 220k-genomic fragment, 1298 bp of a linear double-stranded DNA fragment including oriC (Cm-oriC/220k fragment, SEQ ID NO: 60) was ued. The upstream end region of this joining fragment was homologous to the downstream end of the 220k-genomic fragment (that is, the 60 bases at the upstream end of this fragment consisted of the same base sequence as the 60 bases at the downstream end), and the downstream end region of the joining fragment was homologous to the upstream end of the 220k-genomic fragment (that is, the 60 bases at the downstream end of this fragment consisted of the same base sequence as the upstream 60 bases). Furthermore, the reaction mixture having the composition shown in Table 1 was used as the RCR amplification reaction solution.

Specifically, the Xba I digest of E. coli genomic DNA (DGF-298/XbaI, 4.8ng/µL) and the Cm-oriC/325k fragment having a homologous region with the 325k-genomic fragment that was the target genomic fragment (240pM) were added to the RA reaction [20mM Tris-HCl (pH8.0), 4mM DTT, 150mM KOAc, 10mM Mg(OAc)₂, 100µM ATP, 5% by mass PEG8000, 40mU/µL exonuclease III, 1µM the F203W mutant of E. coli RecA] (5µL), and the solution was incubated at 30°C for 60 minutes to perform the joining reaction. 0.5 µL of the obtained RA product was added to the RCR amplification reaction solution (4.5 µL), and the amplification reaction was performed using a temperature cycle (One cycle of 37°C for 1 minute and then 24°C for 30 minutes is repeated for 40 cycles.). The joining reaction was performed in the same manner using the Cm-oriC/220k fragment instead of the Cm-oriC/325k fragment and setting the target genomic fragment to 220kbp, and then the RCR amplification reaction was performed. As a control, a 200 kbp circular oriC plasmid was similarly subjected to RCR amplification. 50µM of diethylenetriaminepentaacetic acid was added to the RCR amplification reaction solution of the 325k-genomic fragment joining product for long-chain DNA stabilization.

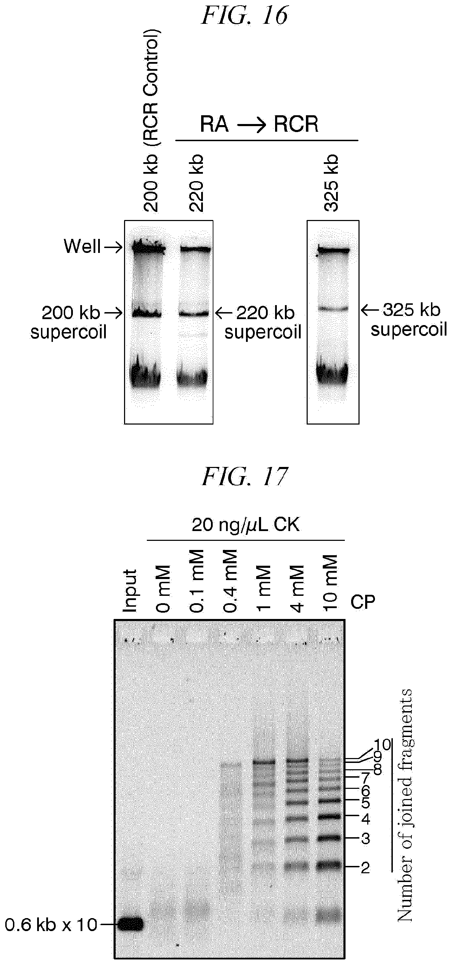

1µL of the reaction mixture after the reaction was subjected to agarose gel electrophoresis, and the separated bands were stained with SYBR Green. The staining results are shown in FIG 16. In the figure, the "220 kb" indicates the lane in which the amplification product obtained by the reaction with a target genomic fragment of 220kbp was run. "325 kbp" indicates the lane in which the amplification product obtained by the reaction with a target genomic fragment of 325 kbp was run. "200 kb (RCR Control)" indicates a lane in which a product obtained by directly amplifying a 200 kb circular oriC plasmid was run. As a result, a supercoiled form of 220 kbp of a circular joined body and a supercoiled form of 325 kbp of a circular joined body were detected the reaction with the target genomic fragment of 220 kbp and in the reaction with the target genomic fragment of 325 kbp, respectively. From these results, it was confirmed that a double-stranded DNA fragment as long as 325 kbp can be cyclized by the DNA production method according to the present invention.

### [Example 15]

Two or more types of linear double-stranded DNA fragments were joined using RecA family recombinase protein and 3'→ 5' exonuclease. In the reaction, the effects of creatine phosphate concentration in the reaction solution containing the ATP regeneration system consisting of creatine kinase and creatine phosphate were investigated.

As the linear double-stranded DNA fragments to be joined, DCW1 to DCW10 (SEQ ID NO: 1 to SEQ ID NO: 10) were used. The F203W mutant of E. coli RecA was used as the RecA family recombinase protein, and exonuclease III was used as the 3'→ 5' exonuclease.

Specifically, first, the reaction solutions consisting of 2nM each of DCW1 to DCW10, 1.5µM of the F203W mutant of RecA, 80mU/µL of exonuclease III, 20mM of Tris-HCl (pH8.0), 4mM of DTT, 1mM of magnesium acetate, 50mM of potassium glutamate, 100µM of ATP, 150mM of TMAC, 5% by mass of PEG 8000, 10% by volume of DMSO, 20ng/µL of creatine kinase, and 0mM (no addition), 0.1mM, 0.4mM, 1mM, 4mM, or 10mM creatine phosphate were prepared. Next, these reaction solutions were incubated at 42°C for 30 minutes to perform the joining reaction, then incubated at 65°C for 2 minutes for heat treatment, and then rapidly cooled on ice. After the heat treatment and rapid cooling, 1.5µL of the reaction solutions were subjected to agarose gel electrophoresis, and the separated bands were stained with SYBR Green.

The staining results are shown in FIG 17. In the figure, "Input" indicates the lane in which 2 µL of the solution containing 2 nM each of DCW1 to DCW10 was run. As a result, it was detected that among the samples subjected to the joining reaction, a band of the joined body obtained by joining all 10 types of fragments in the samples with 0.4 to 10 mM of creatine phosphate. In particular, it was found that the samples with 1 mM or 4 mM of creatine phosphate had a large amount of 10-fragment joined body, and among these samples, the sample with 4 mM of creatine phosphate had also a large amount of 2 to 9-fragment joined bodies and was excellent in the joining efficiency.

### [Example 16]

Two or more types of linear double-stranded DNA fragments were joined using RecA family recombinase protein and 3'→ 5' exonuclease. In the reaction, the effects of creatine kinase concentration in the reaction solution containing the ATP regeneration system consisting of creatine kinase and creatine phosphate were investigated.

As the linear double-stranded DNA fragments to be joined, DCW1 to DCW10 (SEQ ID NO: 1 to SEQ ID NO: 10) were used. The F203W mutant of E. coli RecA was used as the RecA family recombinase protein, and exonuclease III was used as the 3'→ 5' exonuclease.

Specifically, first, the reaction solutions consisting of 2 nM each of DCW1 to DCW10, 1.5 µM of the F203W mutant of RecA, 80 mU/µL of exonuclease III, 20 mM of Tris-HCl (pH8.0), 4 mM of DTT, 1mM of magnesium acetate, 50 mM of potassium glutamate, 100 µM of ATP, 150 mM of TMAC, 5 % by mass of PEG 8000, 10 % by volume of DMSO, 4 mM creatine phosphate and 0 ng/µL, 20 ng/µL, 50 ng/µL or 200 ng/µL of creatine kinase were prepared. Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5 µL of the reaction solutions were subjected to agarose gel electrophoresis, and the separated bands were stained with SYBR Green.

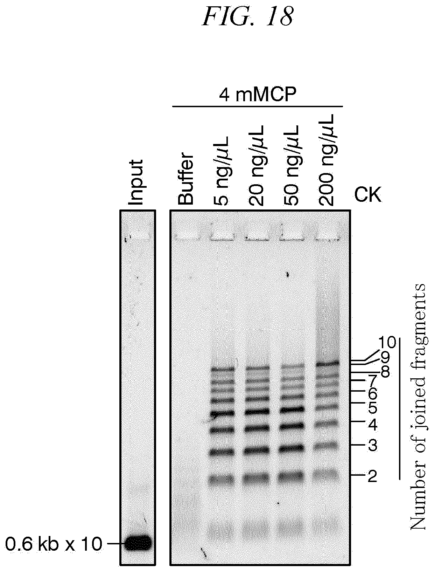

The staining results are shown in FIG 18. In the figure, the "Input" indicates the lane in which 2 µL of the solution containing 2 nM each of DCW1 to DCW10 was run. "Buffer" indicates the lane in which the sample with no creatine kinase added (0 ng/µL) was run. As a result, among the samples subjected to the joining reaction, a band of the joined body obtained by joining all 10 types of fragments was detected in all samples with creatine kinase added.

### [Example 17]

Two or more types of linear double-stranded DNA fragments were joined using RecA family recombinase protein and 3'→ 5' exonuclease. In the reaction, the effects of the ATP regeneration system consisting of pyruvate kinase and phosphoenolpyruvate were investigated.

As the linear double-stranded DNA fragments to be joined, the set of DCW1 to DCW35 (SEQ ID NO: 1 to SEQ ID NO: 35) and Km-oriC (DCW35) (SEQ ID NO: 53) which contained oriC and a pair of the ter sequences inserted outwardly with respect to oriC respectively was used. TheF203W mutant of E. coli RecA was used as the RecA family recombinase protein, and exonuclease III was used as the 3'→ 5' exonuclease.

Specifically, first, the reaction solutions consisting of 0.6nM each of DCW1 to DCW35 (SEQ ID NO: 1 to SEQ ID NO: 35) and Km-oriC (DCW35) (SEQ ID NO: 53), 1.5 µM of the F203W mutant of RecA, 80 mU/µL of exonuclease III, 20 mM of Tris-HCl (pH8.0), 4 mM of DTT, 1 mM of magnesium acetate, 50 mM of potassium glutamate, 100 µM of ATP, 150 mM of TMAC, 5 % by mass of PEG 8000, 10 % by volume of DMSO, 2 mM of phosphoenolpyruvate, and 10 ng/µL, 32 ng/µL, or 100 ng/µL pyruvate kinase were prepared. A reaction solution for comparison was prepared in the same manner except that 2 mM creatine phosphate was substituted for 2 mM phosphoenolpyruvate and 20 ng/µL creatine kinase was mixed instead of pyruvate kinase. A reaction solution for comparison was also prepared in the same manner except that phosphoenolpyruvate and pyruvate kinase were not included. Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5 µL of the reaction solutions were subjected to agarose gel electrophoresis, and the separated bands were stained with SYBR Green.

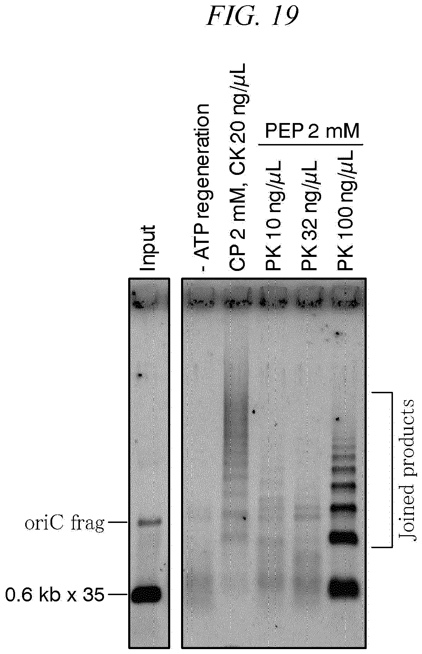

The staining results are shown in FIG 19. In the figure, the "Input" indicates the lane in which 2 µL of the solution containing 0.6 nM each of DCW1 to DCW35 (SEQ ID NO: 1 to SEQ ID NO: 35) and Km-oriC (DCW35) was run. "-ATP regeneration" indicates the lane in which the sample without phosphoenolpyruvate and pyruvate kinase was run. "CP 2 mM, CK 20 ng/µL" indicates the lane in which the sample with creatine phosphate and creatine kinase was run. "PEP 2 mM" indicates the lane in which the sample with phosphoenolpyruvate and pyruvate kinase was run. As a result, the bands of joined bodies with multiple fragments were detected in the samples containing the ATP regeneration system consisting of 2 mM phosphoenolpyruvate and 100 ng/µL pyruvate kinase, similar to the samples containing the ATP regeneration system consisting of creatine phosphate and creatine kinase.

### [Example 18]

Two or more types of linear double-stranded DNA fragments were joined using RecA family recombinase protein and 3'→ 5' exonuclease. In the reaction, the effects of the ATP regeneration system consisting of polyphosphate kinase and polyphosphate were investigated.

As the linear double-stranded DNA fragments to be joined, DCW1 to DCW10 (SEQ ID NO: 1 to SEQ ID NO: 10) were used. The F203W mutant of E. coli RecA was used as the RecA family recombinase protein, and exonuclease III was used as the 3'→ 5' exonuclease.

Specifically, first, the reaction solutions consisting of 2 nM each of DCW1 to DCW10, 1 µM of the wild-type of RecA, 80 mU/µL of exonuclease III, 20 mM of Tris-HCl (pH8.0), 4 mM of DTT, 1 mM of magnesium acetate, 50 mM of potassium glutamate, 100 µM of ATP, 150 mM of TMAC, 5 % by mass of PEG 8000, 10 % by volume of DMSO, 1 mM, 4 mM, or 10 mM of polyphosphate and 20 ng/µL, 60 ng/µL, or 150 ng/µL of polyphosphate kinase were prepared. Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5 µL of the reaction solutions were subjected to agarose gel electrophoresis, and the separated bands were stained with SYBR Green.

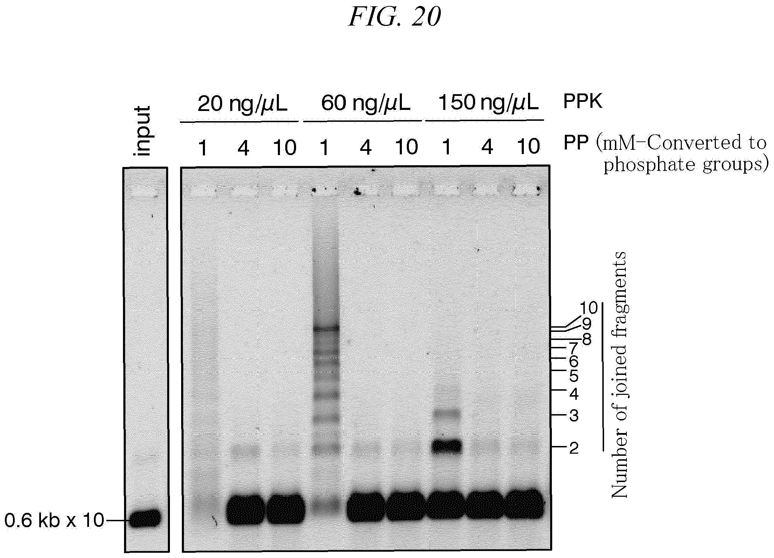

The staining results are shown in FIG 20. In the figure, the "Input" indicates the lane in which 2 µL of the solution containing 2 nM each of DCW1 to DCW10 was run. As a result, among the samples subjected to the joining reaction, a band of the joined body obtained by joining all 10 types of fragments in the sample with 60 ng/µL of polyphosphate kinase and 1mM of polyphosphate.

### [Example 19]

Two or more types of linear double-stranded DNA fragments were joined using RecA family recombinase protein and 3'→ 5' exonuclease. In the reaction, the effect of using a combination of a linear double-stranded DNA-specific 3'→ 5' exonuclease and a single-stranded DNA-specific 3'→ 5' exonuclease was investigated.

As the linear double-stranded DNA fragments to be joined, DCW1 to DCW10 (SEQ ID NO: 1 to SEQ ID NO: 10) were used. The wild-type of E. coli RecA was used as the RecA family recombinase protein. As the linear double-stranded DNA specific 3'→ 5' exonuclease and the single-stranded DNA specific 3'→ 5' exonuclease, exonuclease III and exonuclease I were used respectively.

Specifically, first, the reaction solutions consisting of 1 nM each of DCW1 to DCW10, 1 µM of the wild-type of RecA, 80 mU/µL of exonuclease III, 20 mM of Tris-HCl (pH8.0), 4 mM of DTT, 1 mM of magnesium acetate, 50mM of potassium glutamate, 100 µM of ATP,150 mM of TMAC, 5 % by mass of PEG 8000, 10 % by volume of DMSO, 1 mM, 4 mM, or 10 mM of polyphosphate and 20 ng/µL, 60 ng/µL, or 150 ng/µL of polyphosphate kinase were prepared. Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5 µL of the reaction solutions were subjected to agarose gel electrophoresis, and the separated bands were stained with SYBR Green.

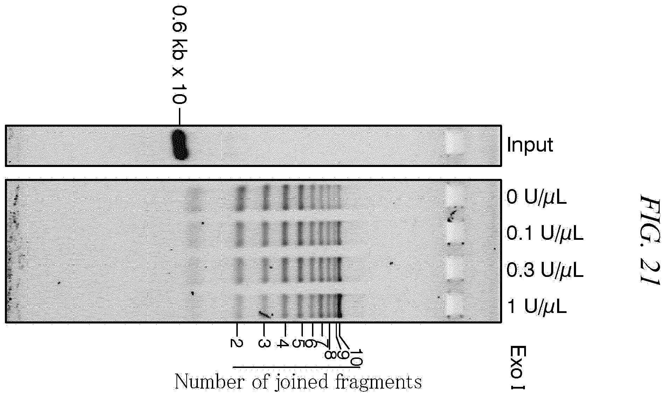

The staining results are shown in FIG 21. In the figure, the "Input" indicates the lane in which 2 µL of the solution containing 1 nM each of DCW1 to DCW10 was run. As a result, in all samples subjected to the joining reaction, a band of the joined body obtained by joining all 10 types of fragments. The amount of the joined body obtained by joining all 10 types of fragments was increased depending on the exonuclease I content. From these results, it was found that addition of exonuclease I promotes joining reaction using exonuclease III and RecA.

### [Example 20]

36 types of linear double-stranded DNA fragments were joined using RecA family recombinase protein, linear double-stranded DNA-specific 3'→ 5' exonuclease, and single-stranded DNA-specific 3'→ 5' exonuclease to form a circular joined body, and the circular joined body was RCR amplified.

As the linear double-stranded DNA fragments to be joined, the set of DCW1 to DCW35 (SEQ ID NO: 1 to SEQ ID NO: 35) and Km-oriC (DCW35) (SEQ ID NO: 52) which contained oriC and a pair of the ter sequences inserted outwardly with respect to oriC respectively was used. The wild-type of E. coli RecA was used as the RecA family recombinase protein. As the linear double-stranded DNA specific 3'→ 5' exonuclease and the single-stranded DNA specific 3'→ 5' exonuclease, exonuclease III and exonuclease I were used respectively. Furthermore, as the RCR amplification reaction solution, a mixture solution containing 60nM of Tus in the reaction mixture having the composition shown in Table 1 was used.

Specifically, first, the reaction solutions consisting of 0.6 nM each of DCW1 to DCW35 and Km-oriC(DCW35), 1 µM of the wild-type of RecA, 80 mU/µL of exonuclease III, 20 mM of Tris-HCl (pH8.0), 4 mM of DTT, 1 mM of magnesium acetate, 50 mM of potassium glutamate, 100µM of ATP, 150mM of TMAC, 5% by mass of PEG 8000, 10% by volume of DMSO, 20 ng/µL of creatine kinase, 4 mM of creatine phosphate, and 0 U/µL (no addition), 0.3 U/µL, or 1 U/µL of exonuclease I were prepared. Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5µL of the reaction solutions were subjected to agarose gel electrophoresis, and the separated bands were stained with SYBR Green.

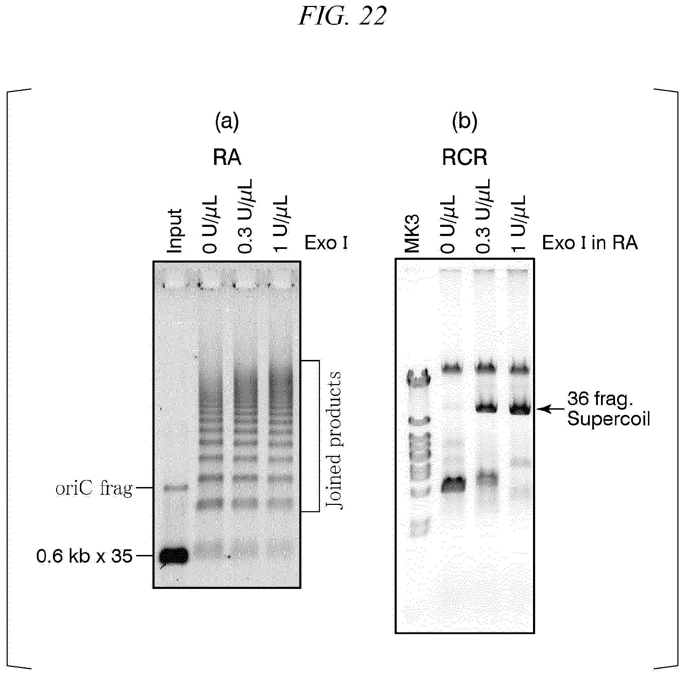

The staining results are shown in FIG 22 (a). In the figure, "Input" indicates the lane in which 2 µL of the solution containing 0.6 nM each of DCW1 to DCW35 and Km-oriC (DCW35) was run. As shown in FIG 22 (a), multi-fragment joined bodies were detected in all samples. The sample with a larger amount of exonuclease I added had a larger amount of multi-fragment joined bodies.

Next, reaction mixtures were prepared by adding 0.5 µL of the reaction solution after the heat treatment and rapid cooling to 4.5 µL of the RCR amplification reaction solution. The reaction mixtures were incubated at 30 °C for 16 hours to perform the RCR amplification. Subsequently, 0.5 µL of each RCR amplification reaction product was diluted to 4 µL by the reaction buffer, which was obtained by removing only the enzyme group from the reaction mixture shown in Table 1, and then re-incubated at 30 °C for 30 minutes. 2.5 µL of the reaction mixture after the re-incubation was subjected to agarose gel electrophoresis, and the separated bands were stained with SYBR Green.

The staining results are shown in FIG 22 (b). As a result, in the sample with exonuclease I, a band of a supercoiled form of a circular 36-fragment joined body ("36-frag scDNA" in the figure) was detected ("36 frag. Supercoil" in FIG 13 (b)). On the other hand, this band was not detected in the sample without exonuclease I. From these results, it was found that the joining efficiency of the joining reaction using RecA and exonuclease III was promoted by the addition of exonuclease I, resulting in a circular joined body obtained by joining all 36 types of fragments.

### [Example 21]

50 types of linear double-stranded DNA fragments were joined using RecA family recombinase protein, 3'→ 5' exonuclease, and single-stranded DNA-specific 3'→ 5' exonuclease to form a circular joined body, and the circular joined body was RCR amplified.

As the linear double-stranded DNA fragments to be joined, the set of DCW1 to DCW49 (SEQ ID NO: 1 to SEQ ID NO: 49) and Km-oriC (DCW49) (SEQ ID NO: 62) which contained oriC and a pair of the ter sequences inserted outwardly with respect to oriC respectively was used. Km-oriC (DCW49) was a linear double-stranded DNA fragment of 1509 bp, and the region of 60 bases from the first to the 60^{th} was the homologous region for joining with DCW49, which consisted of the same base sequence as 60 bases from 532^{rd} to 591^{st} of DCW49. The region of 60 bases from 1450^{th} to 1509^{th} of Km-oriC (DCW49) was the region for joining with DCW1, which consisted of the same base sequence as 60 bases from the first to the 60^{th} of DCW1. That is, when all 50 fragments of DCW1 to DCW49 and Km-oriC (DCW49) were joined, circular DNA was obtained.

As a positive control, the set of DCW1 to DCW35 (SEQ ID NO: 1 to SEQ ID NO: 35) and Km-oriC (DCW35) which contained oriC and a pair of the ter sequences inserted outwardly with respect to oriC respectively was used. The wild-type of E. coli RecA was used as the RecA family recombinase protein. As the linear double-stranded DNA specific 3'→ 5' exonuclease and the single-stranded DNA specific 3'→ 5' exonuclease, exonuclease III and exonuclease I were used respectively. Furthermore, as the RCR amplification reaction solution, a mixture solution containing 60nM of Tus in the reaction mixture having the composition shown in Table 1 was used.

Specifically, first, the reaction solutions consisting of 0.6 nM each of DCW1 to DCW49 and Km-oriC(DCW49), 1 µM of the wild-type of RecA, 80 mU/µL of exonuclease III, 20 mM of Tris-HCl (pH8.0), 4 mM of DTT, 1 mM of magnesium acetate, 50 mM of potassium glutamate, 100 µM of ATP, 150 mM of TMAC, 5 % by mass of PEG 8000, 10 % by volume of DMSO, 20 ng/µL of creatine kinase, 4 mM of creatine phosphate, and 0.3 U/µL of exonuclease I were prepared. A reaction solution prepared in the same manner except that 0.6 nM each of DCW1 to DCW35 and Km-oriC (DCW35) were mixed instead of 0.6 nM each of DCW1 to DCW49 and Km-oriC (DCW49). Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5 µL of the reaction solutions were subjected to agarose gel electrophoresis, and the separated bands were stained with SYBR Green.

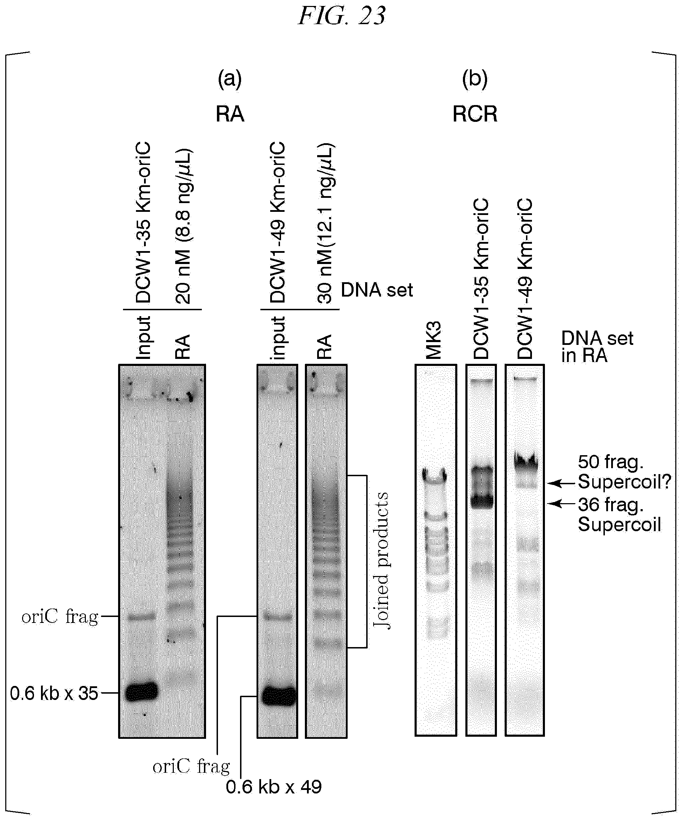

The staining results are shown in FIG 23 (a). In the figure, among "DCW1-35 Km-oriC 20 nM (8.8 ng/mL)", "Input" indicates the lane in which 1.5 µL of the solution containing 0.6 nM each of DCW1 to DCW35 and Km-oriC (DCW35) was run, and the "RA" indicates the lane in which the reaction solution containing 0.6 nM each of DCW1 to DCW35 and Km-oriC (DCW35) was run. Among the "DCW1-49 Km-oriC 30 nM (12.1 ng/mL)", the "Input" indicates the lane in which 1.5 µL of the solution containing 0.6 nM each of DCW1 to DCW49 and Km-oriC (DCW49) was run, and the "RA" indicates the lane in which the reaction solution containing 0.6 nM each of DCW1 to DCW49 and Km-oriC (DCW49) was run. As a result, multi-fragment joined bodies were detected in both samples using DCW1-DCW35 and Km-oriC (DCW35) and samples using DCW1-DCW49 and Km-oriC (DCW49).

Next, reaction mixtures were prepared by adding 0.5 µL of the reaction solution after the heat treatment and rapid cooling to 4.5 µL of the RCR amplification reaction solution. The reaction mixtures were incubated at 30 °C for 16 hours to perform the RCR amplification. Subsequently, 0.5 µL of each RCR amplification reaction product was diluted to 4 µL by the reaction buffer, which was obtained by removing only the enzyme group from the reaction mixture shown in Table 1, and then re-incubated at 30 °C for 30 minutes. 2.5 µL of the reaction mixture after the re-incubation was subjected to agarose gel electrophoresis, and the separated bands were stained with SYBR Green.

The staining result are shown in FIG 23 (b). In the figure, "MK3" indicates the lane in which the DNA ladder marker was run. As a result, as confirmed in Example 20, in the sample using DCW1 to DCW35 and Km-oriC (DCW35), a circular joined body of 36 fragments was obtained and amplification products of this was dected. On the other hand, in the sample using DCW1 to DCW49 and Km-oriC (DCW49), a thin band was detected at the position where a band of a circular joined body of 50 fragments was expected.

Next, DNA contained in the reaction solution obtained by the RCR amplification reaction after the joining reaction using the reaction solution containing DCW1-DCW49 and Km-oriC (DCW49) (amplified product of a circular joined body obtained by joining 50 fragments) was isolated, and the base sequence structure of the DNA was examined.

Specifically, 9 µL of TE buffer (a solution containing 10 mM Tris-HCl (pH 8.0) and 1mM EDTA) was added to 1 µL of the solution after the RCR reaction, and 1 µL of the obtained diluted solution mixed with 50 µL of a solution containing E. coli competent cells (E. coli HST08 Premium Electro-Cells, manufactured by Takara Bio Inc.). The resulting mixture was electroporated and transformed. Twelve colonies of the obtained transformants were cultured overnight in 20 mL of LB liquid medium containing 50 µg/mL kanamycin, and plasmid DNA retained in Escherichia coli cells grown in each culture solution were extracted.. The DNA concentration of the obtained DNA extract was calculated by measuring the absorbance thereof at a wavelength of 260 nm. Based on the calculated DNA concentration, 15 ng of the extracted DNA was subjected to agarose gel electrophoresis, and the separated bands were stained with SYBR Green.

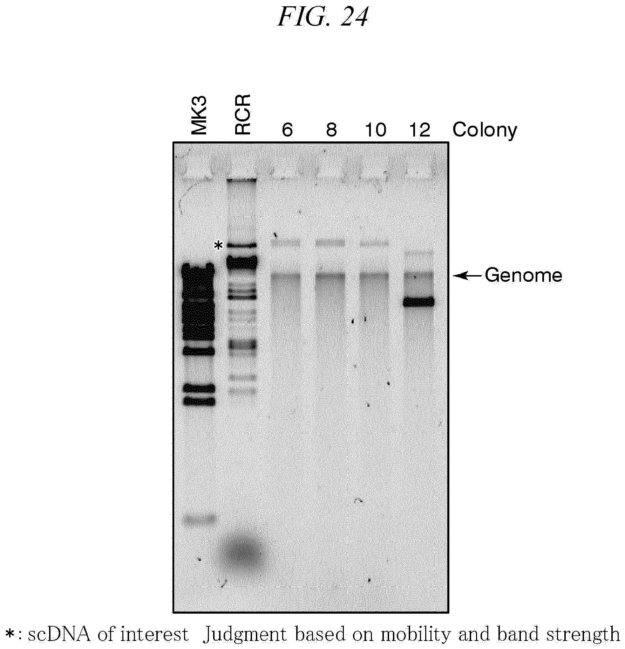

As a result, in 3 colonies (No. 6, 8, 10) of the 12 colonies, a band of the amplification product of the 50-fragment joined body (double-stranded circular DNA without gaps or nicks) was detected. Next, about these 3 colonies and the colony (No. 12) in which the band of the amplification product of the 50-fragment joined body could not be detected, 15 ng of the extracted DNA was subjected to agarose gel electrophoresis using a gel consisting of 1% by mass agarose, and the separated bands were stained with SYBR Green. The staining result is shown in FIG 24. In the figure, the "MK3" indicates the lane in which the DNA ladder marker was run, and the "RCR" indicates the lane in which the the solution after the RCR reaction was run. The "genome" indicates the band of E. coli genomic DNA, and the "*" indicates the band of the 50-fragment joined body. As shown in FIG 24, in the transformants constituting 6, 8, and 10 colonies, only the bands of the amplification products of the Escherichia coli genomic DNA and the 50-fragment joined body were detected.

Next, the sequence structures of the target DNA assumed to be circular joined bodies obtained by j oinig 50 fragments obtained from transformants of No. 6, 8, and 10 colonies were examined. Based on the nucleotide sequence of this 50-fragment joined body, it was revealed that digestion with the restriction enzyme PciI gave a total of four fragments of 10,849 bp, 8,121 bp, 4,771 bp, and 3,694 bp, and digestion with the restriction enzyme NcoI ave a total of six fragments of fragments of 11,308 bp, 7,741 bp, 4,407 bp, 2,599 bp, 1,123 bp, and 257 bp. Therefore, the target DNA assumed to be a circular joined body obtained from each transformant was digested with PciI or NcoI, and their band patterns were examined.

Specifically, 0.5 µL of 0.03 ng/µL of the extracted DNA was added to 4.5 µL of the RCR amplification reaction solution to prepare a reaction solution. The reaction solution was incubated at 30 °C for 16 hours to perform the RCR amplification reaction. Subsequently, 5 µL of each RCR amplification reaction product was diluted to be 20 µL of the RCR reaction buffer (the "reaction buffer" in Table 1), and then re-incubated at 30 °C for 30 minutes. 25 µL of the reaction mixture after the re-incubation was added to 25 µL of a solution containing 50 mM of Tris-HCl (pH 8.0), 50 mM of EDTA, 0. 2% by weight of sodium dodecyl sulfate, 100 µg/mL of Pronase K, 10 % by weight of glycerol, and 0.2 % by weight of bromophenol blue, and then incubated at 37 °C for 30 minutes to decompose the RCR reaction proteins. An equal amount of PCI solution (TE saturated phenol: chloroform: isoamyl alcohol = 25: 24: 1) was added to the solution after the incubation, and the mixture was vigorously mixed using a vortex mixer, and then centrifuged at 12000 rpm for 1 minute. The separated aqueous layer was dialyzed against TE buffer using MF (trademark) -Membrane Filters (Filter Type: 0.05 µm VMWP, manufactured by Merck). The DNA concentration of the DNA solution after the dialysis was calculated based on the absorbance of the DNA solution at a wavelength of 260 nm. 4.5 µL of a solution containing 40 ng of the DNA after the dialysis, 1 × NEBuffer 3 and 0.1 % by mass of BSA was prepared, and 0.5 µL of 10 U/µL of the restriction enzyme PciI (manufactured by Takara Bio Inc.), 10 U/µL of the restriction enzyme NcoI (manufactured by New England Biolab) or water was added to the solution. The resulting solution was incubated at 37 °C for 30 minutes. 2.5 µL of the reaction solution after the incubation was subjected to agarose gel electrophoresis, and the separated bands were stained with SYBR Green.

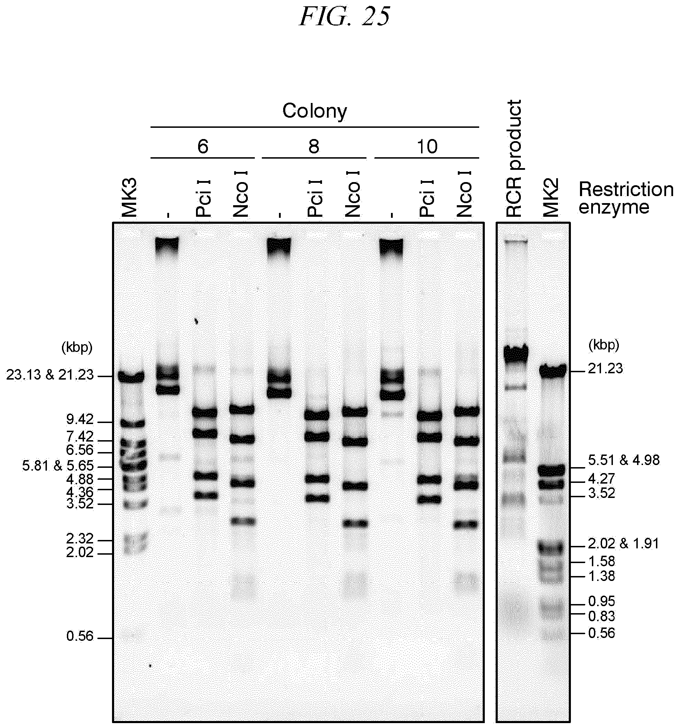

The staining result is shown in FIG 25. In the figure, the "MK3" and the "MK2" indicate the lanes in which the DNA ladder marker were run, respectively. The "PCR product" indicates the lane in which the solution after the RCR reaction was run. The "6", "8", and "10" indicate the lanes in which the RCR amplification reaction products of DNA extracted from transformants of 6, 8, and 10 colonies were run, respectively. The "-" indicates the lane in which the sample without enzyme treatment was run. From the result, it was confirmed that the circular DNA contained in No.6, 8, and 10 transformants were the circular joined bodies in which 50 fragments of interest were joined, based on their band patterns of the digests of PciI and NcoI.

### [Example 22]

Two or more types of linear double-stranded DNA fragments containing DNA fragment with the homologous region at or near 3'- protruding end were joined using RecA family recombinase protein and 3'→ 5' exonuclease. In the reaction, the effect of using a combination of a linear double-stranded DNA-specific 3'→ 5' exonuclease and a single-stranded DNA-specific 3'→ 5' exonuclease in the reaction was investigated.

As the linear double-stranded DNA fragments to be joined, the linear double-stranded DNA fragment with 3'- protruding ends which was obtained by digesting pUC4KSceI (PUC4KSceI fragment) and the linear double-stranded DNA fragment designed to be joined to form a circular joined body with this linear double-stranded DNA fragment (Km-oriC PI-SceI) were used. The pUC4KSceI was a plasmid prepared by joining and circularizing the 4 kbp-fragment and the 500 bp- PI-SceI fragment (SEQ ID NO: 65) with RA. The 4 kbp-fragment was obtained by the PCR amplification using a pUC4K plasmid as a template, and a primer pair (CTATGCGGCATCAGAGCAG (SEQ ID NO: 63) and GTTAAGCCAGCCCCGACAC (SEQ ID NO: 64)). The Km-oriC PI-SceI was the PCR fragment amplified using Km-oriC (DCW35) fragment as a template and a primer pair ((tgcgtaagcggggcacatttcattacctctttctccgcacGCTCTGCCAGTGTTACAACC (SEQ ID NO: 66) and taatgtatactatacgaagttattatctatgtcgggtgcTAACGCGGTATGAAAATGGAT (SEQ ID NO: 67)).

The F203W mutant of E. coli RecA was used as the RecA family recombinase protein. As the linear double-stranded DNA specific 3'→ 5' exonuclease and the single-stranded DNA specific 3'→ 5' exonuclease, exonuclease III and exonuclease I were used respectively.

Specifically, first, 1.28 nM each of pUC4KSceI fragment and Km-oriC PI-SceI, 1.5 µM of the wild-type of RecA, 80 mU/µL of exonuclease III, 20 mM of Tris-HCl (pH8.0), 4 mM of DTT, 1 mM of magnesium acetate, 50 mM of potassium glutamate, 100 µM of ATP, 150 mM of TMAC, 5 % by mass of PEG 8000, 10 % by volume of DMSO, 4 mM of creatine phosphate, 20 ng/µL of creatine kinase, and 0 U/µL (no addition), 0.3 U µL, 0.6 U/µL, or 1 U/µL of exonuclease I were prepared. Next, these reaction solutions were incubated at 42 °C for 60 minutes to perform the joining reaction, then incubated at 65 °C for 5 minutes for heat treatment, and then rapidly cooled on ice. After the heat treatment and rapid cooling, 1.5 µL of the reaction solutions were subjected to agarose gel electrophoresis, and the separated bands were stained with SYBR Green.

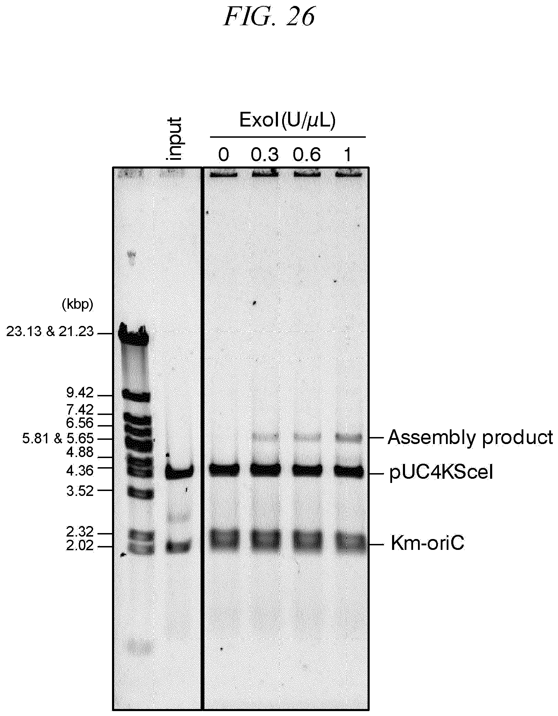

The staining results are shown in FIG 26. In the figure, the "Input" indicates the lane in which 2 µL of the solution containing 1.28 nM each of the pUC4KSceI fragment and Km-oriC PI-SceI was run. In the figure, the "pUC4KSceI" indicates the band of the pUC4KSceI fragment, the "Km-oriC" indicates the band of the Km-oriC PI-SceI, and the "Assembly product" indicates the band of the joined body obtained by joining the pUC4KSceI fragment and the Km-oriC PI-SceI. As a result, the sample with a larger amount of exonuclease I added had a larger amount of fragment joined bodies. The reason was considered that the joining efficiency was increased by exonuclease I digesting 3'- protruding ends, which were difficult to be targeted by Exonuclease III, to turn into a 5'- protruding ends, which were easily targeted by exonuclease III.

### [Example 23]

Two or more types of linear double-stranded DNA fragments joined using RecA family recombinase protein and 3'→ 5' exonuclease. In the reaction, the effect of using a combination of a linear double-stranded DNA-specific 3'→ 5' exonuclease and two types of single-stranded DNA-specific 3'→ 5' exonucleases was investigated.

As the linear double-stranded DNA fragments to be joined, DCW34 to DCW43 (SEQ ID NO: 34 to SEQ ID NO: 43) were used. The wild-type of E. coli RecA was used as the RecA family recombinase protein. Exonuclease III was used as the linear double-stranded DNA-specific 3'→ 5' exonuclease, and exonuclease I and exonuclease T were used as the single-stranded DNA-specific 3'→ 5' exonuclease.

Specifically, first, the reaction solutions consisting of 1 nM each of DCW34 to DCW43, 1 µM of the wild-type of RecA, 80 mU/µL of exonuclease III, 1 U/µL of exonuclease I, 20 mM of Tris-HCl (pH8.0), 4 mM of DTT, 1 mM of magnesium acetate, 50 mM of potassium glutamate, 100 µM of ATP, 150 mM of TMAC, 5 % by mass of PEG 8000, 10 % by volume of DMSO, 20 ng/µL of creatine kinase, 4 mM of creatine phosphate, and 0 U/µL (no addition), 0.05 U µL, 0.15 U/µL, or 0.5 U/µL of exonuclease T were prepared. Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5µL of the reaction solutions were subjected to agarose gel electrophoresis, and the separated bands were stained with SYBR Green.

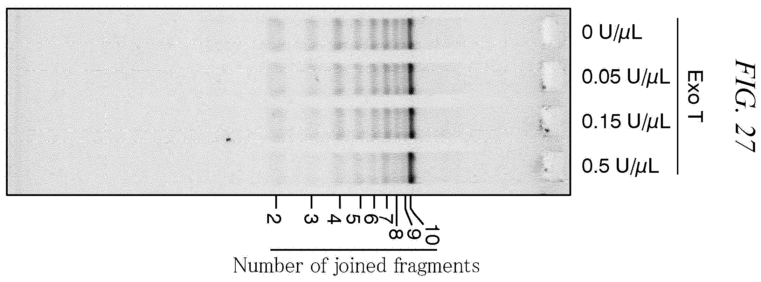

The staining results are shown in FIG 27. As a result, in all samples subjected to the joining reaction, a band of the joined body obtained by joining all 10 types of fragments. The amount of 2 to 9-fragment joined bodies was decreased depending on the exonuclease T content. From these results, it was found that addition of exonuclease I and exonuclease T promoted the reaction of joining many joining fragments.

### [Example 24]

Two or more types of linear double-stranded DNA fragments joined using RecA family recombinase protein and 3'→ 5' exonuclease. Bacteriophage RecA homolog T4 phage UvsX was used as the RecA family recombinase protein. DCW34 to DCW43 (SEQ ID NO: 34 to SEQ ID NO: 43) were used as linear double-stranded DNA fragments to be joined.

Specifically, first, the reaction solutions consisting of 1 nM each of DCW34 to DCW43, 8 mU/µL, 30 mU/µL, or 80 mU/µL of exonuclease III, 1 U/µL of exonuclease I, 20 mM of Tris-HCl (pH8.0), 4 mM of DTT, 1 mM of magnesium acetate, 50 mM of potassium glutamate, 100 µM of ATP, 150 mM of TMAC, 5 % by mass of PEG 8000, 10 % by volume of DMSO, 20 ng/µL of creatine kinase, 4 mM of creatine phosphate, and, 0 µM (no addition), 1 µM, or 3 µM of UvsX or 1 µM of the wild-type of RecA (Control) were prepared. Next, these reaction solutions were incubated at 42 °C for 30 minutes to perform the joining reaction, then incubated at 65 °C for 2 minutes for heat treatment, and then rapidly cooled on ice. After the heat treatment and rapid cooling, 1.5 µL of the reaction solutions were subjected to agarose gel electrophoresis, and the separated bands were stained with SYBR Green.

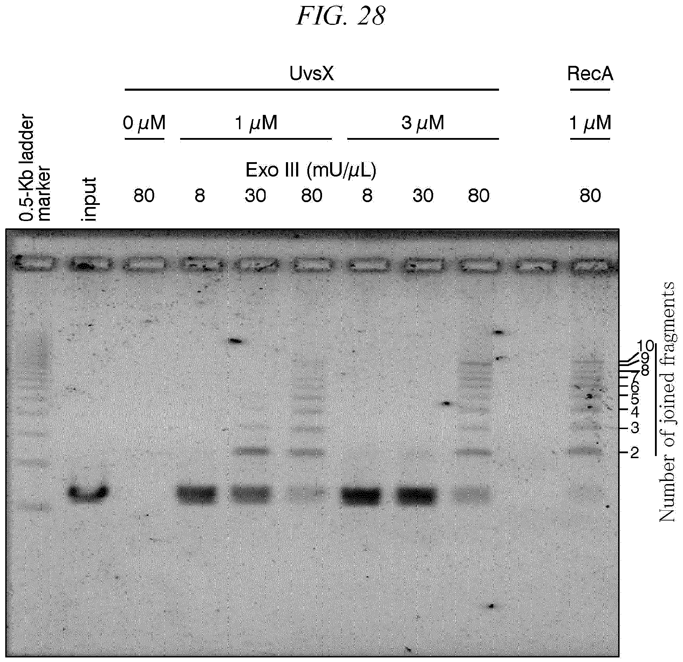

The staining results are shown in FIG 28. In the figure, the "Input" indicates the lane in which 2 µL of the solution containing 1 nM each of DCW34 to DCW43 was run. As a result, a band of the joined body obtained by joining all 10 types of fragments in the samples performed the joining reaction in the presence of 1 µM or 3 µM UvsX and 80 mU/µL exonuclease III, similar in the samples performed the joining reaction in the presence of 1µM RecA wild-type and 80 mU/µL exonuclease III. From these results, it was confirmed that the joining reaction can be performed with a high joining efficiency even when UvsX, which was a bacteriophage RecA homolog, was used, as when RecA was used.

### [Example 25]

In the reaction of j oinng two or more types of linear double-stranded DNA fragments using UvsX and 3'→ 5' exonuclease, the effect of using T4 phage UvsY together was investigated. DCW34 to DCW43 (SEQ ID NO: 34 to SEQ ID NO: 43) were used as linear double-stranded DNA fragments to be joined.

Specifically, first, the reaction solutions consisting of 1 nM each of DCW34 to DCW43, 3 µM of UvsX, 60 mU/µL of exonuclease III, 1 U/µL of exonuclease I, 20 mM of Tris-HCl (pH8.0), 4 mM of DTT, 1 mM of magnesium acetate, 50 mM of potassium glutamate, 100 µM of ATP, 150 mM of TMAC, 5 % by mass of PEG 8000, 10 % by volume of DMSO, 20 ng/µL of creatine kinase, 4 mM of creatine phosphate, and, 0 µM (no addition), 0.1 µM, 0.3 µM, or 1 µM of UvsY were prepared. Next, these reaction solutions were incubated at 42°C for 30 minutes to perform the joining reaction, then incubated at 65 °C for 2 minutes for heat treatment, and then rapidly cooled on ice. After the heat treatment and rapid cooling, 1.5 µL of the reaction solutions were subjected to agarose gel electrophoresis, and the separated bands were stained with SYBR Green.

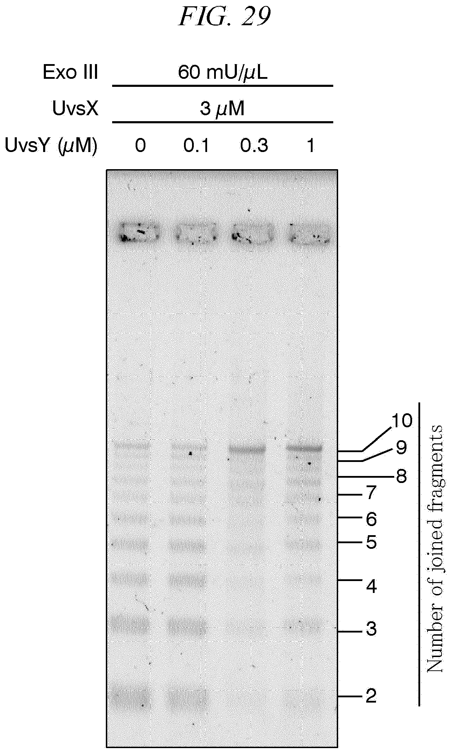

The staining results are shown in FIG 29. As a result, in all samples subjected to the joining reaction, a band of the joined body obtained by joining all 10 types of fragments. Depending on the UvsY content, the amount of the joined body obtained by joining all 10 types of fragments was increased and the amount of 2 to 9-fragment joined bodies was decreased. From these results, it was found that using UvsX and UvsY together promoted the joining reaction using exonuclease III and UvsX is promoted.

### [Explanation of symbols]

1a,1b•••linear double-stranded DNA fragment, H•••homologous region, 2 ...3'→ 5' exonuclease, 3... RecA family recombinase protein.

## Claims

1. A DNA production method, the method comprising:
preparing a reaction solution containing two or more types of DNA fragments, a protein having RecA family recombinase activity and a linear double-stranded DNA-specific 3'→5' exonuclease, and
producing linear or circular DNA in the reaction solution by joining the two or more types of DNA fragments to each other at regions having homologous base sequences or regions having complementary base sequences.

2. The DNA production method according to Claim 1,
wherein the reaction solution further contains a linear double-stranded DNA-specific 3'→5' exonuclease and single-stranded DNA-specific 3'→5' exonuclease.

3. The DNA production method according to any one of Claims 1 to 2,
wherein the reaction solution contains a regenerating enzyme for nucleoside triphosphates or deoxynucleotide triphosphates and its substrate.

4. The DNA production method according to any one of Claims 1 to 3,
wherein the joining reaction of the two or more types of DNA fragments is performed within a temperature range of 25 to 48 °C.

5. The DNA production method according to any one of Claims 1 to 4,
wherein linear or circular DNA is obtained by joining 7 or more DNA fragments.

6. The DNA production method according to any one of Claims 1 to 5,
wherein the reaction solution contains one or more selected from the group consisting of tetramethylammonium chloride and dimethyl sulfoxide.

7. The DNA production method according to any one of Claims 1 to 6,
wherein the protein having RecA family recombinase activity is uvsX, and the reaction solution further contains uvsY.

8. The DNA production method according to any one of Claims 1 to 7,
wherein the reaction solution at the start of the joining reaction of the two or more types of DNA fragments contains two or more types of DNA fragments with the same molar concentration.

9. The DNA production method according to any one of Claims 1 to 8,
further comprising repairing gaps and nicks in the obtained linear or circular DNA using gap repair enzymes.

10. The DNA production method according to Claim 9,
further comprising heat-treating the obtained linear or circular DNA at 50 to 70 °C, followed by rapidly cooling it to 10 °C or lower, and then repairing the gaps and nicks using gap repair enzymes.

11. The DNA production method according to any one of Claims 1 to 8,
wherein the DNA obtained by joining is linear, and
performing PCR using the linear DNA directly as a template.

12. The DNA production method according to any one of Claims 1 to 8,
wherein the DNA obtained by joining is a circular DNA containing a replication origin sequence capable of binding to an enzyme having DnaA activity, and
forming a reaction mixture which contains the circular DNA, a first enzyme group that catalyzes replication of circular DNA, a second enzyme group that catalyzes an Okazaki fragment joining reaction and synthesizes two sister circular DNAs constituting a catenane, a third enzyme group that catalyzes a separation of two sister circular DNAs, and dNTP.

13. The DNA production method according to any one of Claims 1 to 8,
further comprising introducing the obtained linear or circular DNA into a microorganism, and amplifying the double-stranded DNA with gaps and nicks repaired.

14. A DNA fragment-joining kit, comprising:
a protein having RecA family recombinase activity and a linear double-stranded DNA-specific 3'→5' exonuclease,
wherein the kit is used for producing linear or circular DNA by joining two or more types of DNA fragments to each other at regions having homologous base sequences or regions having complementary base sequences.

## Patentansprüche

1. DNA-Herstellungsverfahren, wobei das Verfahren Folgendes umfasst:
Herstellen einer Reaktionslösung, die zwei oder mehr Typen von DNA-Fragmenten, ein Protein mit Rekombinaseaktivität der RecA-Familie und eine lineare doppelsträngige DNA-spezifische 3'→5' Exonuklease enthält, und
Herstellen von linearer oder zirkulärer DNA in der Reaktionslösung durch Miteinanderverbinden der zwei oder mehr Typen von DNA-Fragmenten an Regionen mit homologen Basensequenzen oder Regionen mit komplementären Basensequenzen.

2. DNA-Herstellungsverfahren nach Anspruch 1,
wobei die Reaktionslösung ferner eine lineare doppelsträngige DNA-spezifische 3'→5' Exonuklease und eine einzelsträngige DNA-spezifische 3'→5' Exonuklease enthält.

3. DNA-Herstellungsverfahren nach einem der Ansprüche 1 bis 2,
wobei die Reaktionslösung ein Regenerationsenzym für Nucleosidtriphosphate oder Desoxynucleotidtriphosphate und dessen Substrat enthält.

4. DNA-Herstellungsverfahren nach einem der Ansprüche 1 bis 3,
wobei die Verbindungsreaktion der zwei oder mehr Typen von DNA-Fragmenten in einem Temperaturbereich von 25 bis 48 °C durchgeführt wird.

5. DNA-Herstellungsverfahren nach einem der Ansprüche 1 bis 4,
wobei lineare oder zirkuläre DNA durch Verbinden von 7 oder mehr DNA-Fragmenten erhalten wird.

6. DNA-Herstellungsverfahren nach einem der Ansprüche 1 bis 5,
wobei die Reaktionslösung eines oder mehrere aus der Gruppe bestehend aus Tetramethylammoniumchlorid und Dimethylsulfoxid enthält.

7. DNA-Herstellungsverfahren nach einem der Ansprüche 1 bis 6,
wobei das Protein mit Rekombinaseaktivität der RecA-Familie uvsX ist und die Reaktionslösung ferner uvsY enthält.

8. DNA-Herstellungsverfahren nach einem der Ansprüche 1 bis 7,
wobei die Reaktionslösung zu Beginn der Verbindungsreaktion der zwei oder mehr Typen von DNA-Fragmenten zwei oder mehr Typen von DNA-Fragmenten mit derselben Stoffmengenkonzentration enthält.

9. DNA-Herstellungsverfahren nach einem der Ansprüche 1 bis 8,
das ferner das Reparieren von Lücken und Kerben in der erhaltenen linearen oder zirkulären DNA unter Verwendung von Lückenreparaturenzymen umfasst.

10. DNA-Herstellungsverfahren nach Anspruch 9,
das ferner die Wärmebehandlung der erhaltenen linearen oder zirkulären DNA bei 50 bis 70 °C, gefolgt von seiner raschen Abkühlung auf 10 °C oder weniger, und dann das Reparieren der Lücken und Kerben unter Verwendung von Lückenreparaturenzymen umfasst.

11. DNA-Herstellungsverfahren nach einem der Ansprüche 1 bis 8,
wobei die durch Verbinden erhaltene DNA linear ist, und
das eine PCR unter Verwendung der linearen DNA direkt als Matrize durchführt.

12. DNA-Herstellungsverfahren nach einem der Ansprüche 1 bis 8,
wobei die durch Verbinden erhaltene DNA eine zirkuläre DNA ist, die eine Replikationsursprungssequenz enthält, die sich an ein Enzym mit DnaA-Aktivität binden kann, und
das ein Reaktionsgemisch bildet, das die zirkuläre DNA, eine erste Enzymgruppe, die die Replikation der zirkulären DNA katalysiert, eine zweite Enzymgruppe, die eine Okazaki-Fragmentverbindungsreaktion katalysiert und zwei zirkuläre Schwester-DNAs synthetisiert, die ein Catenan darstellen, eine dritte Enzymgruppe, die eine Trennung von zwei zirkulären Schwester-DNAs katalysiert, und dNTP enthält.

13. DNA-Herstellungsverfahren nach einem der Ansprüche 1 bis 8,
das ferner das Einbringen der erhaltenen linearen oder zirkulären DNA in einen Mikroorganismus und das Amplifizieren der doppelsträngigen DNA mit reparierten Lücken und Kerben umfasst.

14. DNA-Fragment-Verbindungskit, der Folgendes umfasst:
ein Protein mit Rekombinaseaktivität der RecA-Familie und eine lineare doppelsträngige DNA-spezifische 3'→5' Exonuklease,
wobei der Kit zur Herstellung von linearer oder zirkulärer DNA durch Miteinanderverbinden von zwei oder mehr Typen von DNA-Fragmenten an Regionen mit homologen Basensequenzen oder Regionen mit komplementären Basensequenzen verwendet wird.

## Revendications

1. Procédé de production d'ADN, le procédé comprenant :
la préparation d'une solution de réaction qui contient deux types de fragments d'ADN ou plus, une protéine qui présente une activité recombinase de la famille RecA et une exonucléase 3'→5' spécifique à l'ADN à deux brins linéaire ; et
la production d'ADN linéaire ou circulaire dans la solution de réaction en joignant les deux types de fragments d'ADN ou plus les uns aux autres au niveau de régions comportant des séquences de base homologues ou de régions comportant des séquences de base complémentaires.

2. Procédé de production d'ADN selon la revendication 1,
dans lequel la solution de réaction contient en outre une exonucléase 3'→5' spécifique à l'ADN à deux brins linéaire et une exonucléase 3'→5' spécifique à l'ADN à un seul brin.

3. Procédé de production d'ADN selon l'une quelconque des revendications 1 à 2,
dans lequel la solution de réaction contient une enzyme régénératrice pour les triphosphates de nucléoside ou les triphosphates de désoxynucléotide et son substrat.

4. Procédé de production d'ADN selon l'une quelconque des revendications 1 à 3,
dans lequel la réaction de jonction des deux types de fragments d'ADN ou plus est réalisée dans une plage de températures allant de 25 à 48 °C.

5. Procédé de production d'ADN selon l'une quelconque des revendications 1 à 4,
dans lequel de l'ADN linéaire ou circulaire est obtenu en joignant 7 fragments d'ADN ou plus.

6. Procédé de production d'ADN selon l'une quelconque des revendications 1 à 5,
dans lequel la solution de réaction contient un ou plusieurs composés parmi le groupe comprenant le chlorure de tétraméthylammonium et le sulfoxyde de diméthyle.

7. Procédé de production d'ADN selon l'une quelconque des revendications 1 à 6,
dans lequel la protéine qui présente une activité recombinase de la famille RecA est uvsX, et la solution de réaction contient en outre uvsY.

8. Procédé de production d'ADN selon l'une quelconque des revendications 1 à 7,
dans lequel la solution de réaction au début de la réaction de jonction des deux types de fragments d'ADN ou plus contient deux types de fragments d'ADN ou plus qui présentent la même concentration molaire.

9. Procédé de production d'ADN selon l'une quelconque des revendications 1 à 8,
comprenant en outre la réparation de lacunes et d'entailles dans l'ADN linéaire ou circulaire obtenu en utilisant des enzymes de réparation de lacunes.

10. Procédé de production d'ADN selon la revendication 9,
comprenant en outre le traitement thermique de l'ADN linéaire ou circulaire obtenu entre 50 et 70 °C, suivi par son refroidissement rapide jusqu'à 10 °C ou moins, puis la réparation des lacunes et des entailles en utilisant des enzymes de réparation de lacunes.

11. Procédé de production d'ADN selon l'une quelconque des revendications 1 à 8,
dans lequel l'ADN obtenu par jonction est linéaire ; et
qui réalise une PCR en utilisant l'ADN linéaire directement comme modèle.

12. Procédé de production d'ADN selon l'une quelconque des revendications 1 à 8,
dans lequel l'ADN obtenu par jonction est un ADN circulaire qui contient une séquence d'origine de réplication capable de se lier à un enzyme qui présente une activité DnaA ; et
qui forme un mélange de réaction qui contient l'ADN circulaire, un premier groupe d'enzymes qui catalyse la réplication de l'ADN circulaire, un deuxième groupe d'enzymes qui catalyse une réaction de jonction de fragments d'Okazaki et qui synthétise deux ADN circulaires frères constituant une caténane, un troisième groupe d'enzymes qui catalyse une séparation de deux ADN circulaires frères, et dNTP.

13. Procédé de production d'ADN selon l'une quelconque des revendications 1 à 8,
comprenant en outre l'introduction de l'ADN linéaire ou circulaire obtenu à l'intérieur d'un micro-organisme et l'amplification de l'ADN à deux brins dont les lacunes et les entailles ont été réparés.

14. Kit de jonction de fragments d'ADN, comprenant :
une protéine qui présente une activité recombinase de la famille RecA et une exonucléase 3'→5' spécifique à l'ADN à deux brins linéaire,
dans lequel le kit est utilisé pour produire de l'ADN linéaire ou circulaire en joignant deux types de fragments d'ADN ou plus les uns aux autres au niveau de régions comportant des séquences de base homologues ou de régions comportant des séquences de base complémentaires.
